(19) **Europäisches Patentamt / European Patent Office / Office européen des brevets**

(11) **EP 2 162 206 B1**

(12) **EUROPÄISCHE PATENTSCHRIFT**

(45) Veröffentlichungstag und Bekanntmachung des Hinweises auf die Patenterteilung:
**13.04.2011 Patentblatt 2011/15**

(51) Int Cl.:
*B01J 8/00* (2006.01)   *B07B 1/46* (2006.01)

(21) Anmeldenummer: **08760888.1**

(22) Anmeldetag: **12.06.2008**

(86) Internationale Anmeldenummer:
**PCT/EP2008/057342**

(87) Internationale Veröffentlichungsnummer:
**WO 2008/152080 (18.12.2008 Gazette 2008/51)**

(54) **VERFAHREN ZUM EINBRINGEN EINER WENIGSTENS EINER PRODUKTIONSCHARGE VON RINGFÖRMIGEN SCHALENKATALYSATOREN K ENTNOMMENEN TEILMENGE IN EIN REAKTIONSROHR EINES ROHRBÜNDELREAKTORS**

METHOD FOR INTRODUCING INTO A REACTION TUBE OF A TUBE BUNDLE REACTOR, A PARTIAL AMOUNT WITHDRAWN FROM AT LEAST ONE PRODUCTION CHARGE OF ANNULAR-SHAPED SHELL CATALYSTS K

PROCÉDÉ POUR INTRODUIRE DANS UN TUBE À RÉACTION D'UN RÉACTEUR À FAISCEAUX DE TUBES, UNE QUANTITÉ PARTIELLE PRÉLEVÉE D'AU MOINS UNE CHARGE DE PRODUCTION DE CATALYSEURS À ENVELOPPE ANNULAIRES K

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**

(30) Priorität: **15.06.2007 DE 102007028333**
**15.06.2007 US 944327 P**

(43) Veröffentlichungstag der Anmeldung:
**17.03.2010 Patentblatt 2010/11**

(73) Patentinhaber: **BASF SE**
**67056 Ludwigshafen (DE)**

(72) Erfinder:
• **CREMER, Ulrich**
**68161 Mannheim (DE)**
• **WILMER, Hagen**
**67071 Ludwigshafen (DE)**
• **RAICHLE, Andreas**
**67063 Ludwigshafen (DE)**
• **PETERSEN, Hermann**
**67269 Grünstadt (DE)**
• **BORCHERT, Holger**
**67591 Offstein (DE)**
• **STRAHBERGER, Horst**
**67125 Dannstadt-schauernheim (DE)**
• **MÜLLER-ENGEL, Klaus, Joachim**
**76297 Stutensee (DE)**

(56) Entgegenhaltungen:
**EP-A- 0 700 866    DE-A1- 10 047 693**
**GB-A- 778 134**

**Beschreibung**

Vorliegende Erfindung betrifft ein Verfahren zum Einbringen einer wenigstens einer Produktionscharge von ringförmigen Schalenkatalysatoren K entnommenen Teilmenge in ein Reaktionsrohr eines Rohrbündelreaktors zum Zweck der Beschickung dieses Reaktionsrohres mit einem zur Durchführung einer heterogen katalysierten partiellen Gasphasenoxidation einer organischen Ausgangsverbindung geeigneten Katalysatorfestbett.

Verfahren der heterogen katalysierten partiellen Gasphasenoxidation von organischen Ausgangsverbindungen in in den Reaktionsrohren von Rohrbündelreaktoren befindlichen Katalysatorfestbetten sind zur Herstellung zahlreicher Industriechemikalien bekannt.

Als solche heterogen katalysierte partielle Gasphasenoxidationen organischer Verbindungen seien beispielhaft genannt die Umsetzung von Methanol zu Formaldehyd (vgl. z.B. CH-A 449 600 und CH-A 38 828), die Umsetzung von Propen zu Acrolein und/oder Acrylsäure (vgl. z.B. die DE-A 23 51 151), die Umsetzung von tert.-Butanol, iso-Buten, iso-Butan, iso-Butyraldehyd oder dem Methylether des tert.-Butanols zu Methacrolein und/oder Methacrylsäure (vgl. z.B. DE-A 25 26 238, EP-A 092 097, EP-A 058 927, DE-A 41 32 263, DE-A 41 32 684 und DE-A 40 22 212), die Umsetzung von Acrolein zu Acrylsäure sowie von Methacrolein zu Methacrylsäure (vgl. z.B. DE-A 25 26 238), die Umsetzung von o-Xylol und/oder Naphthalin zu Phthalsäureanhydrid (vgl. z.B. EP-A 522 871) sowie die Umsetzung von Butadien zu Maleinsäureanyhdrid (vgl. z.B. DE-A 21 06 796 und DE-A 16 24 921), die Umsetzung von $C_4$-Kohlenwasserstoffen wie 1-Buten, 2-Buten, Butadien und/oder n-Butan zu Maleinsäureanhydrid (vgl. z.B. GB-A 14 64 198 und GB-A 12 91 354), die Umsetzung von Indanen zu Anthrachinonen (vgl. z.B. DE-A 20 25 430), die Umsetzung von Ethylen zu Ethylenoxid (vgl. z.B. EP-A 352 849, EP-A 352 850, EP-A 532 325, US-A 5,155,242 und US-A 5,262,551) oder von Propylen zu Propylenoxid (vgl. z.B. DE-AS 12 54 137, DE-A 21 59 346, EP-A 372 972, WO 89/07101, DE-A 43 11 608), die Umsetzung von Propylen und/oder Acrolein zu Acrylnitril (vgl. z.B. DE-A 23 51 151), die Umsetzung von iso-Buten und/oder Methacrolein zu Methacrylnitril (d.h., der Begriff der partiellen Oxidation soll in dieser Schrift auch die partielle Ammoxidation, d.h., eine partielle Oxidation im Beisein von Ammoniak, umfassen), die oxidative Dehydrierung von Kohlenwasserstoffen oder Kohlenwasserstoffderivaten (vgl. z.B. DE-A 23 51 151), die Umsetzung von Propan zu Acrylnitril oder zu Acrolein und/oder Acrylsäure (vgl. z.B. DE-A 101 31 297, EP-A 10 90 684, EP-A 608 838, DE-A 100 46 672, EP-A 529 853, WO 01/96270 und DE-A 100 28 582) etc..

Während in dieser Schrift unter einer vollständigen Oxidation einer organischen Verbindung mit molekularem Sauerstoff verstanden wird, dass die organische Verbindung unter reaktiver Einwirkung von molekularem Sauerstoff so umgesetzt wird, dass der in der organischen Verbindung insgesamt enthaltene Kohlenstoff in Oxide des Kohlenstoffs und der in der organischen Verbindung insgesamt enthaltene Wasserstoff in Oxide des Wasserstoffs umgewandelt wird, werden in dieser Schrift alle davon verschiedenen exothermen Umsetzungen einer organischen Verbindung unter reaktiver Einwirkung von molekularem Sauerstoff als Partialoxidationen einer organischen Verbindung zusammengefasst.

Im besonderen sollen in dieser Schrift unter Partialoxidationen solche exothermen Umsetzungen organischer Verbindungen unter reaktiver Einwirkung von molekularem Sauerstoff verstanden werden, bei denen die partiell zu oxidierende organische Verbindung nach beendeter Umsetzung wenigstens ein Sauerstoffatom mehr chemisch gebunden enthält, als vor Durchführung der Partialoxidation.

Bei einem Rohrbündelreaktor handelt es sich dabei normalerweise um einen Apparat, der ein vertikal angeordnetes Bündel von Reaktionsrohren umfasst, welches von einem Reaktormantel umschlossen wird, wobei beide Enden der einzelnen Reaktionsrohre offen sind und jedes Reaktionsrohr mit seinem oberen Ende in eine Durchgangsöffnung eines oben in den Reaktormantel eingedichteten oberen Rohrbodens und mit seinem unteren Ende in eine Durchgangsöffnung eines unten in den Reaktormantel eingedichteten unteren Rohrbodens eingedichtet ausläuft, wobei das Äußere der Reaktionsrohre, der obere und der untere Rohrboden, und der Reaktormantel gemeinsam den Reaktionsrohrumgebungsraum abgrenzen, sowie jeder der beiden Rohrböden von einer wenigstens eine Öffnung aufweisenden Reaktorhaube überspannt wird. Bei der Durchführung einer heterogen katalysierten partiellen Gasphasenoxidation in einem solchen Rohrbündelreaktor sind dessen Reaktionsrohre mit einem Katalysatorfestbett beschickt (ist in dessen Reaktionsrohren ein Katalysatorfestbett eingefüllt (eingebracht); befindet sich in dessen Reaktionsrohren ein Katalysatorfestbett) und über die wenigstens eine Öffnung in einer der beiden Reaktorhauben wird ein die partiell zu oxidierende organische Verbindung (organische Ausgangsverbindung) und molekularen Sauerstoff enthaltendes Reaktionsgaseingangsgemisch zu- und über die wenigstens eine Öffnung der anderen Reaktorhaube das durch partielle Gasphasenoxidation der partiell zu oxidierenden organischen Ausgangsverbindung zum gewünschten Zielprodukt beim Durchströmen des in den Reaktionsrohren befindlichen Katalysatorfestbetts resultierendes Zielprodukt enthaltendes Produktgasgemisch abgeführt, während auf der Mantelseite des Rohrbündelreaktors um die Reaktionsrohre wenigstens ein (in der Regel flüssiges) Wärmeaustauschmittel geführt wird. Normalerweise wird im Fall der Verwendung von wenigstens einem flüssigen Wärmeaustauschmittel das selbige so um die Reaktionsrohre geführt, dass jede der beiden einander zugewandten Oberflächen der beiden Rohrböden von flüssigem Wärmeaustauschmittel benetzt wird. Das wenigstens eine (z.B. flüssige) Wärmeaustauschmittel wird üblicherweise mit einer Temperatur $T_w^{ein}$ in den Reaktionsrohrumgebungsraum hinein- und mit der Temperatur $T_w^{aus}$ wieder aus dem Reaktionsrohrumgebungsraum herausgeführt.

Die Aussage, dass die Reaktionsrohre in die Durchgangsöffnungen im oberen bzw. unteren Rohrboden eingedichtet sind, bringt zum Ausdruck, dass zwischen der Reaktionsrohraußenwand und der Bohrungswand (bzw. Wand der Durchgangsöffnung oder auch Einhüllenden der Durchgangsöffnung) keine Durchtrittsmöglichkeit für das Wärmeaustauschmittel besteht. Eine solche Eindichtung kann z.B. wie in der DE- 20 2006 014 116 U1 beschrieben erfolgen.

Grundsätzlich kann das wenigstens eine Wärmeaustauschmittel auch gasförmig oder im Siedezustand befindlich durch den Reaktionsrohrumgebungsraum geführt werden. Beispiele für derartige Rohrbündelreaktoren und in selbigen durchgeführte heterogen katalysierte partielle Gasphasenoxidationen offenbaren z.B. die EP-A 700 893, die DE-A 44 31 949, die WO 03/057 653, die EP-A 16 95 954, die WO 03/055 835, die WO 03/059 857, die WO 03/076 373, die DE 699 15 952 T2, die DE-A 10 2004 018 267, die DE 20 2006 014 116 U1 und das Deutsche Aktenzeichen 10 2007 019 597.6, sowie der in den vorgenannten Schriften zitierte Stand der Technik.

In der Regel sind die Bauteile des Rohrbündelreaktors aus Stahl gefertigt. Dabei kommt als Fertigungsstahl sowohl Edelstahl (z.B. der DIN Werkstoffnummer 1.4541 oder 1.4571) als auch Schwarzstahl bzw. ferritischer Stahl (z.B. die DIN-Werkstoffe 1.0481, 1.0315 oder der Werkstoff 1.0425) in Betracht. Häufig sind alle Bauteile des Rohrbündelreaktors aus der gleichen Stahlsorte gefertigt. Vielfach sind die Reaktorhauben aus ferritischem Stahl gefertigt und auf ihrer Innenseite mit Edelstahl plattiert. Teilweise ist der Reaktormantel auch aus einer anderen Stahlsorte als die übrigen Teile des Rohrbündelreaktors gefertigt, da zu seiner Herstellung gewalzter Stahl verwendet werden kann.

Als Reaktionsrohrumgebungsraum ist in dieser Schrift der durch das Äußere der Reaktionsrohre, die beiden Rohrböden und den Reaktormantel zusammen begrenzte Raum definiert, in welchem das wenigstens eine (in der Regel flüssige) Wärmeaustauschmittel geführt wird. In einfachster Weise wird im Reaktionsrohrumgebungsraum nur ein (vorzugsweise flüssiges) Wärmeaustauschmittel geführt (eine solche Verfahrensweise wird auch als Einzonenfahrweise im Einzonenrohrbündelreaktor bezeichnet). Es wird dem Reaktionsrohrumgebungsraum üblicherweise an seinem oberen oder an seinem unteren Ende mit seiner Eintrittstemperatur $T_w^{ein}$ durch Öffnungen im Reaktormantel zu- und am entgegengesetzten Ende mit einer Austrittstemperatur $T_w^{aus}$ durch Öffnungen im Reaktormantel wieder aus dem Reaktionsrohrumgebungsraum herausgeführt.

Durch die Exothermie von Gasphasenpartialoxidationen bedingt gilt während der Durchführung einer heterogen katalysierten partiellen Gasphasenoxidation $T_w^{aus} \geq T_w^{ein}$ (die Gleichheit bezieht sich auf den Fall einer Siedekühlung). Mit Hilfe eines Wärmeaustauschers wird üblicherweise einer Teil- oder der Gesamtmenge des aus dem Reaktionsrohrumgebungsraum herausgeführten (vorzugsweise flüssigen) Wärmeaustauschmittels Wärme entzogen, bevor es mit der Temperatur $T_w^{ein}$ dem Reaktionsrohrumgebungsraum wieder zugeführt wird.

Im Reaktionsrohrumgebungsraum kann das (vorzugsweise flüssige) Wärmeaustauschmittel grundsätzlich im einfachen Gleich- oder Gegenstrom zum in den Reaktionsrohren strömenden Reaktionsgasgemisch um die Reaktionsrohre geführt werden. Es kann aber auch mit Hilfe entsprechender Umlenkscheiben mäanderförmig um die Reaktionsrohre geführt werden, so dass lediglich über den gesamten Reaktionsrohrumgebungsraum ein Gleich- oder Gegenstrom zur Strömungsrichtung des Reaktionsgasgemischs in den Reaktionsrohren besteht. Ist das verwendete Wärmeaustauschmittel unter den Einsatzbedingungen flüssig, sollte es anwendungstechnisch zweckmäßig einen Schmelzpunkt im Bereich von 0 (oder von 50) bis 250°C, vorzugsweise von 120 bis 200°C aufweisen.

Als solche flüssigen Wärmeaustauschmittel kommen z.B. Schmelzen von Salzen wie Kaliumnitrat, Kaliumnitrit, Natriumnitrit und/oder Natriumnitrat sowie Schmelzen von Metallen wie Kalium, Natrium, Quecksilber und Legierungen verschiedener Metalle in Betracht. Aber auch ionische Flüssigkeiten (in denen wenigstens eines der entgegensetzt geladenen Ionen wenigstens ein Kohlenstoffatom enthält) oder Wärmeträgeröle (z.B. hochsiedende organische Lösungsmittel wie Mischungen aus Diphyl® und Dimethylphthalat) sind einsetzbar. Als gasförmige Wärmeaustauschmittel kommen z.B. unter erhöhtem Druck befindlicher Wasserdampf oder auch Rauchgase in Betracht. Siedekühlung kann z.B. auch mit unter Druck befindlichem Siedewasser vorgenommen werden.

Zur Verbesserung der Selektivität der Zielproduktbildung kann man die heterogen katalysierte partielle Gasphasenoxidation einer organischen Verbindung aber auch als Mehrzonenfahrweise (z.B. Zweizonenfahrweise) in einem Mehrzonenrohrbündelreaktor (z.B. in einem Zweizonenrohrbündelreaktor) ausführen. In diesem Fall werden im Reaktionsrohrumgebungsraum (z.B. zwei) voneinander im wesentlichen räumlich getrennte (vorzugsweise flüssige) Wärmeaustauschmittel (die normalerweise von der selben Sorte sind) geführt (diese können z.B. durch im Reaktionsrohrumgebungsraum eingezogene und für die Reaktionsrohre entsprechende Durchtrittsöffnungen aufweisende Trennrohrböden separiert sein).

Der Reaktionsrohrlängsabschnitt, über den sich das jeweilige (vorzugsweise flüssige) Wärmeaustauschmittel erstreckt, repräsentiert eine Temperatur- bzw. Reaktionszone (der Einzonenrohrbündelreaktor weist in entsprechender Weise nur eine Reaktionszone auf).

Innerhalb der jeweiligen Temperaturzone kann das (vorzugsweise flüssige) Wärmeaustauschmittel wie bei der Einzonenfahrweise geführt werden (auch relativ zur Strömungsrichtung des Reaktionsgasgemischs). Für den Unterschied zwischen $T_w^{aus}$ und $T_w^{ein}$ gilt die einzelne Temperaturzone betreffend das bezüglich der Einzonenfahrweise Gesagte in im wesentlichen gleicher Weise.

Eine graphische Unterscheidung zwischen einer Einzonen- und einer Zweizonenfahrweise (zwischen einem Einzonenrohrbündelreaktor und einem Zweizonenrohrbündelreaktor) zeigen schematisch z. B. die Figuren des Deutschen Aktenzeichens 102007019597.6 sowie die Figuren der EP-A 1695954. Mehrzonenfahrweisen sind im übrigen z. B. beschrieben in den Schriften EP-A 1734030, DE-A 10313214, DE-A 10313219, DE-A 10313211, DE-A 10313208 sowie in dem in diesen Schriften zitierten Stand der Technik. Sie sind vor allem dann vorteilhaft, wenn eine hohe Belastung des Katalysatorfestbetts mit der partiell zu oxidierenden organischen Verbindung gewählt wird. Unter der Belastung des Katalysatorfestbetts mit Reaktionsgasgemisch oder mit einer Reaktionsgasgemischkomponente wird die Menge an Reaktionsgasgemisch bzw. Reaktionsgasgemischkomponente in Normlitern (NI; das Volumen, das die entsprechende Menge rechnerisch bei 0 °C und 1 atm gasförmig einnehmen würde) verstanden, die pro Stunde durch einen Liter Katalysatorfestbett (reine Inertschüttungen werden nicht miteinbezogen) geführt wird.

Die Temperatur $T_w^{ein}$ des wenigstens einen (vorzugsweise flüssigen) Wärmeaustauschmittels liegt bei heterogen katalysierten partiellen Gasphasenoxidationen organischer Ausgangsverbindungen in typischer Weise im Bereich von 200 bis 500°C, häufig im Bereich von 250 bis 400°C und vielfach im Bereich von 250 bis 310°C.

Der Arbeitsdruck kann bei einer heterogen katalysierten partiellen Gasphasenoxidation sowohl unterhalb von Normaldruck (z.B. bis zu 0,5 bar, das Reaktionsgasgemisch wird durchgesaugt) als auch oberhalb von Normaldruck liegen. Typischerweise wird der vorgenannte Arbeitsdruck bei Werten von 1 bis 5 bar, häufig 1,5 bis 3,5 bar (jeweils absolut) liegen. Normalerweise wird der Arbeitsdruck bei einer heterogen katalysierten partiellen Gasphasenoxidation einer organischen Ausgangsverbindung 100 bar nicht überschreiten.

Das Reaktionsgaseingangsgemisch (bzw. auch Reaktionsgaseintrittsgemisch) selbst kann bei den unterschiedlichen Verfahrensweisen im Rohrbündelreaktor in den Reaktionsrohren sowohl von oben nach unten als auch von unten nach oben geführt werden (d. h., die wenigstens eine Zuführöffnung kann sich entweder in der oberen oder in der unteren Reaktorhaube befinden). Das gleiche trifft auf die Führung des (vorzugsweise flüssigen) Wärmeaustauschmittels zu.

Das Reaktionsgaseingangsgemisch kann bei seinem Eintritt in die Reaktionsrohre grundsätzlich auf die Temperatur des auf der entsprechenden Rohrbodenunterseite strömenden Wärmeaustauschmittels vorerwärmt sein.

Die Temperatur des Reaktionsgaseintrittsgemischs kann sich bei dessen Eintritt in die Reaktionsrohre aber auch unterhalb dieser Temperatur des Wärmeaustauschmittels befinden. Dies ist dann sinnvoll, wenn die Reaktionsrohre in Strömungsrichtung des Reaktionsgasgemischs zunächst mit einem Längsabschnitt aus gegenüber der Partialoxidation inerten Formkörpern beschickt sind, bevor der katalytisch aktive (wirksame) Abschnitt des Katalysatorfestbetts mit katalytisch wirksame Aktivmasse aufweisenden Formkörpern beginnt. Beim Durchströmen dieses inerten Abschnitts kann sich das Reaktionsgaseintrittsgemisch dann auf die Temperatur des den entsprechenden katalytisch aktiven Reaktionsrohrabschnitt umströmenden Wärmeaustauschmittels erwärmen. Prinzipiell kann das Reaktionsgaseingangsgemisch (das Produktgasgemisch) über mehr als eine in der entsprechenden Reaktorhaube befindliche Zuführöffnung (Abführöffnung) zugeführt (abgeführt) werden. In der Regel erfolgen aber sowohl die Zufuhr des Reaktionsgaseintrittsgemischs als auch die Abfuhr des Produktgasgemischs jeweils über nur eine Öffnung in der entsprechenden Reaktorhaube.

Häufig kann eine heterogen katalysierte partielle Gasphasenoxidation einer organischen Verbindung räumlich unmittelbar einer heterogen katalysierten partiellen Gasphasenoxidation einer anderen organischen Verbindung nachgeschaltet (in diesem Fall ist das Zielprodukt der vorausgehenden Partialoxidation normalerweise die partiell zu oxidierende organische Verbindung in der nachgeschalteten Partialoxidation) oder vorgeschaltet erfolgen. Insbesondere in diesen Fällen kann die zuführende bzw. abführende Reaktorhaube zu einer zylindrischen Rohröffnung reduziert (als zylindrische Rohröffnung ausgeführt) sein, die z. B. einen zylindrischen Übergang zu einem Nachkühler bilden kann (vgl. z. B. DE-A 10 2004 018267 und Deutsches Aktenzeichen 102007019597.6).

Selbstredend können auch zwei heterogen katalysierte partielle Gasphasenoxidationen, die zwei aufeinanderfolgende Gasphasenpartialoxidationsschritte abbilden, unmittelbar aufeinanderfolgend in den Reaktionszonen eines Mehrzonenrohrbündelreaktors (z. B. in einem Zweizonenrohrbündelreaktor) durchgeführt werden, wobei sich dabei die Beschickung des Katalysatorfestbetts in den Reaktionsrohren des Mehrzonenrohrbündelreaktors beim Übergang vom einen Reaktionsschritt zum nachfolgenden Reaktionsschritt normalerweise in entsprechender Weise ändert (vgl. z. B. die Durchführung mehrstufiger heterogen katalysierter partieller Gasphasenoxidationen im sogenannten "single reactor" gemäß EP-A 1388533, US-A 6069271, EP-A 990636, US-A 2006/0161019 und EP-A 1106598). Beispiele für die Durchführung solcher mehrstufiger heterogen katalysierter partieller Gasphasenoxidationen im Mehrzonenrohrbündelreaktor (z. B. Zweizonenrohrbündelreaktor) sind die heterogen katalysierte partielle Gasphasenoxidation von Propylen zu Acrylsäure sowie von iso-Buten zu Methacrylsäure.

Neben molekularem Sauerstoff und der partiell zu oxidierenden organischen Ausgangsverbindung als Reaktanden enthält das Reaktionsgaseingangsgemisch einer heterogen katalysierten partiellen Gasphasenoxidation in der Regel noch ein sich unter den Bedingungen der heterogen katalysierten Gasphasenpartialoxidation im wesentlichen inert verhaltendes Verdünnungsgas. Darunter werden in dieser Schrift solche Verdünnungsgase verstanden, deren Bestandteile im Reaktionsgasgemisch befindlich unter den Bedingungen der heterogen katalysierten partiellen Gasphasenoxidation - jeder Bestandteil für sich betrachtet - zu mehr als 95 mol-%, vorzugsweise zu mehr als 99 mol-% unverändert erhalten bleiben. Ihnen kommt die Aufgabe zu, einerseits einen Teil der Reaktionswärme aufzunehmen und als Bestandteil des Produktgasgemischs aus dem Rohrbündelreaktor herauszuführen und andererseits zu gewährleisten, dass sich das Reaktionsgasgemisch in der Regel außerhalb des explosionsfähigen Bereichs befindet. Für heterogen katalysierte partielle Gasphasenoxidationen organischer Ausgangsverbindungen in typischer Weise geeignete inerte Verdünnungsgase sind z.B. $N_2$, $CO_2$, Wasserdampf, Edelgase und vielfach auch gesättigte Kohlenwasserstoffe (z.B. bei einer Partialoxidation von ungesättigten organischen Verbindungen) oder Gemische aus allen oder aus Teilmengen der vorgenannten möglichen inerten Verdünnungsgase.

Die Umsetzung der im Reaktionsgasgemisch einer heterogenen katalysierten partiellen Gasphasenoxidation enthaltenen Reaktanden ($O_2$ und die organische Ausgangsverbindung) erfolgt beim Hindurchführen des Reaktionsgasgemischs durch das in den Reaktionsrohren befindliche Katalysatorfestbett während der Verweilzeit der Reaktanden an der Katalysatoroberfläche.

Die Reaktionsrohre im Rohrbündelreaktor sind, wie bereits erwähnt, in der Regel aus ferritischem Stahl oder aus Edelstahl gefertigt und weisen häufig eine Wanddicke von einigen mm, z.B. 1 bis 3 mm auf. Ihr Innendurchmesser beträgt meist einige cm, z.B. 10 bis 50 mm, häufig 15 bis 30 mm, oder 20 bis 30 mm. Die Rohrlänge erstreckt sich im Normalfall auf wenige Meter (typisch ist eine Reaktionsrohrlänge im Bereich von 1 bis 10 m, häufig 2 bis 8 m oder 2 bis 6 m, vielfach 2 bis 4 m).

Anwendungstechnisch zweckmäßig beläuft sich die im Rohrbündelreaktor untergebrachte Anzahl an Reaktionsrohren auf wenigstens 1000, häufig auf wenigstens 3000 oder 5000 und vielfach auf wenigstens 10000. Häufig beträgt die Anzahl der im Rohrbündelreaktor untergebrachten Reaktionsrohre 15000 bis 30000, oder bis 40000, oder bis 50000. Rohrbündelreaktoren mit einer oberhalb von 50000 liegenden Anzahl von Reaktionsrohren bilden eher die Ausnahme. Innerhalb des Reaktionsrohrumgebungsraums sind die Reaktionsrohre im Normalfall im wesentlichen homogen verteilt angeordnet, wobei die Verteilung zweckmäßig so gewählt wird, dass der Abstand der zentrischen Innenachsen von zueinander nächstliegenden Reaktionsrohren (die sogenannte Reaktionsrohrteilung) 25 bis 55 mm, häufig 35 bis 55 mm beträgt.

Insbesondere bei Rohrbündelreaktoren mit größerem Querschnitt ihrer Rohrböden ist es anwendungstechnisch zweckmäßig, im Zentrum des Rohrbündelreaktors einen unberohrten Bereich zu belassen, und statt dessen in diesem Bereich den oberen Rohrboden abzustützen.

Grundsätzlich unterscheidet man die Gesamtzahl der Reaktionsrohre in Arbeitsrohre (die überwiegende Mehrzahl der Reaktionsrohre) und in Thermorohre. Während die Arbeitsrohre diejenigen Reaktionsrohre sind, in denen die heterogen katalysierte partielle Gasphasenoxidation im eigentlichen Sinn durchgeführt wird, dienen Thermorohre in erster Linie dem Zweck, die Reaktionstemperatur repräsentativ für die anderen Reaktionsrohre (die Arbeitsrohre) zu verfolgen und zu steuern. Zu diesem Zweck enthalten die Thermorohre zusätzlich zum Katalysatorfestbett normalerweise eine lediglich mit einem Temperaturmessfühler (z. B. ein Multithermoelement oder ein axial bewegliches Einfachthermoelement) beschickte, im Thermorohr längs desselben zentriert geführte Thermohülse (dies wird vielfach aber nicht in notwendiger Weise durch einen erhöhten Innendruchmesser der Thermorohre im Vergleich zu den Arbeitsrohren ausgeglichen). Im Regelfall ist die Anzahl der Thermorohre in einem Rohrbündelreaktor sehr viel kleiner als die Anzahl der Arbeitsrohre. Normalerweise beträgt die Anzahl der Thermorohre ≤ 20. Dabei ist es von besonderer Bedeutung, dass die Thermorohre so mit Katalysatorfestbett beschickt werden, dass der Verlauf der Reaktionstemperatur längs des Innern eines Thermorohres dem Verlauf der Reaktionstemperatur längs des Inneren eines Arbeitsrohres möglichst genau entspricht (vgl. EP-A 873 783 und EP-A 1270 065).

Der Verlauf der Reaktionstemperatur in den Reaktionsrohren wird zum einen durch die Wärmeentwicklung bedingt durch die Exothermie einer heterogen katalysierten partiellen Gasphasenoxidation und zum anderen unter anderem durch den Übergang dieser Reaktionswärme auf das wenigstens eine im Reaktionsrohrumgebungsraum geführte Wärmeaustauschmittel bestimmt.

Da heterogen katalysierte partielle Gasphasenoxidationen üblicherweise ausgeprägt exotherme Reaktionen sind, und der Übergang der Reaktionswärme auf das wenigstens eine Wärmeaustauschmittel mit endlicher Geschwindigkeit erfolgt, ist die Temperatur des Reaktionsgasgemischs beim reaktiven Durchgang desselben durch das Katalysatorfestbett von der Temperatur des das Katalysatorfestbett außerhalb der Reaktionsrohre umströmenden fluiden Wärmeaustauschmittels normalerweise verschieden. Sie liegt üblicherweise oberhalb der Eintrittstemperatur des Wärmeaustauschmittels $T_w^{ein}$ in die entsprechende Reaktionszone (Temperaturzone) und durchläuft längs einer Reaktionszone in der Regel ein absolutes Maximum (Heißpunktmaximum) oder fällt von einem absoluten Maximalwert (gegebenenfalls über weitere relative Maxima) ausgehend ab. Diese Maximalwerte der Reaktionstemperatur (der Temperatur des Reaktionsgasgemischs) werden üblicherweise als sogenannte "Heißpunkttemperaturen" bezeichnet.

Die Heißpunkttemperatur ist deshalb von besonderer Bedeutung, weil dort, wo im Reaktionsrohr die Reaktionstemperatur erhöht ist (die Temperatur des Katalysatorfestbetts entspricht im wesentlichen der am jeweiligen Ort herrschenden Temperatur des Reaktionsgasgemischs) auch die irreversiblen Alterungsprozesse im Katalysatorfestbett mit erhöhter Geschwindigkeit verlaufen und eine beschleunigte Deaktivierung des Katalysatorfestbetts bedingen.

Es ist diesbezüglich aus dem Stand der Technik bekannt, dass heterogen katalysierte partielle Gasphasenoxidationen in den mit einem Katalysatorfestbett beschickten Reaktionsrohren eines Rohrbündelreaktors bei sorgfältiger Vorgehensweise über vergleichsweise lange Zeiträume (bis zu mehreren Jahren) durchgeführt werden können, ohne dass das Katalysatorfestbett in den Reaktionsrohren erneuert (frisch beschickt) werden muss (vgl. z.B. DE-A 10 350 822, DE-A 10 2004 025 445, EP-A 17 34 030 und den in diesen Schriften gewürdigten Stand der Technik). Der irreversiblen Deaktivierung des Katalysatorfestbetts wird dabei unter ansonsten im wesentlichen unveränderten Betriebsbedingungen üblicherweise durch eine Erhöhung von $T_w^{ein}$ und/oder eine Erhöhung des Arbeitsdrucks in den Reaktionsrohren entgegengewirkt (vgl. z.B. EP-A 11 06 598, DE-A 10 351 269, EP-A 17 34 030, EP-A 990 636, DE-A 10 2004 025 445). Durch diese Maßnahmen lässt sich die Zielprodukt-Raum-Zeit-Ausbeute über längere Betriebszeiten beibehalten. Allerdings bedingen sie, dass sich der Alterungsprozess des Katalysatorfestbetts zunehmend weiter beschleunigt (bestimmte Bewegungsprozesse innerhalb der Katalysatoren, die zur Alterung beitragen, laufen z.B. zunehmend schneller ab). Bei Erreichen eines Höchstwertes von $T_w^{ein}$ muss das Katalysatorfestbett schließlich vollständig ausgetauscht werden (vgl. auch DE-A 10 232 748, EP-A 11 06 598 und DE-A 10 2007 010 422).

Nachteilig an einem solchen Komplettaustausch ist jedoch, dass er vergleichsweise aufwendig ist. Das Verfahren der Zielproduktherstellung muss für längere Zeit unterbrochen werden und die Kosten der Katalysatorherstellung sind ebenfalls erheblich.

Erwünscht sind daher Verfahrensweisen, die dabei behilflich sind, die Standzeit des Katalysatorfestbetts im Rohrbündelreaktor weitestgehend in die Länge zu ziehen.

Wie bereits erwähnt, ist das Vorgenannte bei sorgfältiger Vorgehensweise in gewissem Umfang möglich. Unter sorgfältiger Vorgehensweise wird dabei im Stand der Technik verstanden, den Rohrbündelreaktor im Rahmen des machbaren insgesamt möglichst so zu betreiben, dass innerhalb der individuellen Reaktionsrohre so weitgehend wie möglich ein einheitliches Reaktionsverhalten und damit auch ein möglichst einheitlicher Verlauf der Reaktionstemperatur (der Temperatur des Reaktionsgemischs bzw. der Temperatur des Katalysatorfestbetts) längs der einzelnen Reaktionsrohre vorliegt.

Die EP-A 14 71 046, die DE-A 20 2006 014 116 U1 und die WO 03/059857 empfehlen diesbezüglich, die heterogen katalysierte partielle Gasphasenoxidation einer organischen Ausgangsverbindung in solchen Rohrbündelreaktoren durchzuführen, deren Reaktionsrohre möglichst einheitlich gefertigt sind.

Gemäß der Lehre der JP-A 2006-142288 soll die Reaktionsrohrinnenoberfläche darüber hinaus eine möglichst niedrige Oberflächenrauhigkeit aufweisen, um eine möglichst einheitliche Beschickung der Reaktionsrohre mit Katalysatorfestbett zu gewährleisten.

Eine solche möglichst einheitliche Beschickung der Reaktionsrohre mit dem selben Katalysatorfestbett empfehlen auch die Schriften US-A 4,701,101, EP-A 14 66 883, WO 03/057653, US-A 2006/245992, US-A 2002/136678, WO 2005/051532, WO 03/076373 und JP-A 2004/195279.

Gleichzeitig wird bei heterogen katalysierten Gasphasenreaktionen ganz generell versucht, den zum Fördern des Reaktionsgases erforderlichen Energieaufwand möglichst niedrig zu halten. Als eine Maßnahme zur Erreichung dieser Zielsetzung werden bevorzugt ringförmige Katalysatorformkörper zur Gestaltung des Katalysatorfestbetts mitverwendet, da diese beim Durchgang des Reaktionsgases durch das Katalysatorfestbett einen besonders geringen Druckverlust bedingen (vgl. z.B. WO 2005/03039). Ein weiterer Vorteil ringförmiger Katalysatorformkörper besteht normalerweise in verringerten Diffusionswegen und daraus resultierend in vielen Fällen in einer verbesserten Zielproduktausbeute.

Im einfachsten Fall besteht ein solcher ringförmiger Katalysatorformkörper nur aus katalytisch aktiver Masse, die gegebenenfalls mit inertem Material (das z.B. vielfach aus Verstärkungsgründen eingearbeitet wird) verdünnt sein kann (gegebenenfalls ist auch noch Formungshilfsmittel enthalten; z.B. Graphit). Solche ringförmigen geometrischen Katalysatorformkörper werden üblicherweise als ringförmige Vollkatalysatoren bezeichnet.

Nachteilig an ringförmigen Vollkatalysatoren ist jedoch deren beim Einfüllen in die Reaktionsrohre in der Regel nicht voll befriedigende mechanische Stabilität. Zwar lässt sich diese durch eine Erhöhung ihrer Wandstärke verbessern, nachteilig an größeren Wandstärken ist jedoch, dass mit ihnen eine Verlängerung des Diffusionsweges aus der Reaktionszone heraus einhergeht, was unerwünschte Folgereaktionen fördert und damit die Zielproduktselektivität mindert.

Eine Auflösung des bei Vollkatalysator-Ringen bestehenden Widerspruchs zwischen erforderlicher mechanischer Stabilität (zunehmender Wandstärke) einerseits und Begrenzung des Diffusionsweges aus der Reaktionszone heraus (abnehmende Wandstärke) andererseits unter Aufrechterhaltung der ansonsten besonders vorteilhaften Ringgeometrie eröffnen ringförmige Schalenkatalysatoren. Dabei handelt es sich um ringförmige Katalysatorformkörper, die aus einem ringförmigen (mechanisch besonders stabilen), bezüglich der Gasphasenpartialoxidation in der Regel inerten (katalytisch nicht aktiven) Trägerformkörper und einer auf dessen Oberfläche aufgebrachten katalytisch aktiven Masse (Aktivmasse) bestehen.

Ihre Herstellung kann z.B. so erfolgen, dass man den ringförmigen (in der Regel aus katalytisch nicht aktivem (häufig oxidischem (z.B. hochgebranntem)) Material bestehenden; aus Inertmaterial bestehenden) Trägerformkörper unter Mitverwendung eines in der Regel flüssigen Bindemittels mit feinteiliger Aktivmasse beschichtet. Alternativ (oder im Gemisch mit feinteiliger Vorläufermasse) können die Trägerformkörper auch mit einer feinteiligen Vorläufermasse der Aktivmasse unter Mitverwendung eines in der Regel flüssigen Bindemittels beschichtet werden und die Überführung in den aktiven ringförmigen Katalysatorformkörper durch nachträgliche (z.B. oxidative und/oder reduktive) thermische Behandlung erfolgen (gegebenenfalls in molekularen Sauerstoff enthaltender Atmosphäre). Das Beschichten kann in einfachster Weise z.B. dadurch erfolgen, dass man die Oberfläche eines inerten ringförmigen Trägerformkörpers (oder auch einfach nur "Trägerkörper") mittels eines flüssigen Bindemittels befeuchtet und nachfolgend feinteilige (pulverförmige) Aktivmasse oder feinteilige (pulverförmige) Vorläufermasse an der befeuchteten Oberfläche anheftet. Nachfolgend wird normalerweise noch wenigstens eine Teilmenge des flüssigen Bindemittels (in der Regel unter Einwirkung von Wärme) verflüchtigt, bevor die ringförmigen Schalenkatalysatoren zum Beschicken eines Reaktionsrohres einbringfertig sind (eine weitere thermische Behandlung kann z.B. innerhalb der Reaktionsrohre befindlich erfolgen (z.B. zum Zweck der Entfernung von restlichem Bindemittel [vgl. z.B. DE-A 102005010645])). Alternativ kann man die ringförmigen Trägerformkörper auch mit einer Suspension von feinteiliger Aktivmasse und/oder feinteiliger Vorläufermasse besprühen.

Anstelle den in der Regel inerten ringförmigen Trägerformkörper mit feinteiliger Aktivmasse oder mit feinteiliger Vorläufermasse zu beschichten, kann man den ringförmigen Trägerformkörper in vielen Fällen aber auch mit einer Lösung (einer molekularen und/oder kolloidalen) der katalytisch aktiven Substanz oder mit einer Lösung einer Vorläufersubstanz tränken und nachfolgend das Lösungsmittel verflüchtigen und gegebenenfalls eine chemische Reduktion und/oder thermische Behandlung (gegebenenfalls in molekularen Sauerstoff enthaltender Atmosphäre) anschließen. Die auf diese Weise resultierenden ringförmigen Katalysatorformkörper werden in der Literatur häufig auch als Träger- oder Tränkkatalysatoren bezeichnet. Sie sollen in dieser Schrift jedoch ebenfalls unter dem Oberbegriff "Schalenkatalysatoren" subsummieren.

Verfahren zur Herstellung von ringförmigen Schalenkatalysatoren, die sich als Katalysatoren für heterogen katalysierte partielle Gasphasenoxidationen eignen, finden sich z. B. beschrieben in den Schriften DE-A 290 9671, EP-A 714 700, Deutsche Anmeldung mit dem Aktenzeichen 102007017080.9, WO 2004/108267, DE 10 2005 010 645 A1, DE-A 103 13 209, DE-A 103 25 488, DE-A 103 60 058, DE-A 103 51 269, DE-A 103 50 822, WO 2007/009922, DE-A 100 49 873, Deutsche Anmeldung mit dem Aktenzeichen 102007010422.9, DE-A 40 06 935, DE-A 198 23 275, DE-A 198 39 001, DE-A 198 23 262, DE-A 103 44 844, US 2006/0205978 und EP-A 758 562 sowie dem in diesen Schriften gewürdigten Stand der Technik.

Ein ungewolltes, in der Regel aber nicht vollständig zu vermeidendes Nebenprodukt bei der Herstellung von ringförmigen Schalenkatalysatoren ist die Ausbildung von "Zwillingen" von ringförmigen Schalenkatalysatoren. Dabei handelt es sich um zwei Schalenkatalysatorringe, die fest aneinander haften. Ihre Entstehung ist letztlich darauf zurückzuführen, dass das zum Aufbringen der Aktivmassenschale auf den ringförmigen Trägerkörper bei der Herstellung von Schalenkatalysatoren üblicherweise mitverwendete, normalerweise flüssige, Bindemittel nicht nur die Verbindung von Aktivmasse und Trägerformkörper zu bewerkstelligen vermag, sondern in begrenztem Umfang auch die ungewollte Verbindung zweier ringförmiger Schalenkatalysatoren erzeugt. Im wesentlichen beschränkt sich die Ausbildung von solchen Zwillingen dabei auf zwei Typen: a) annellierte Zwillinge und b) Tandem-Zwillinge.

Bei den annellierten Zwillingen haften zwei ringförmige Schalenkatalysatoren (ein ringförmiger Schalenkatalysator weist die Geometrie A x I x H (Außendurchmesser x Innendurchmesser x Höhe) auf) mit ihren kreiszylindrischen Einhüllenden (Außenwänden) im wesentlichen über die gesamte Höhe H aneinander. Sie haften quasi auf gleicher Höhe nebeneinander stehend aneinander (ihre Mantelfläche haften aneinander).

Bei den Tandem-Zwillingen haften zwei ringförmige Schalenkatalysatoren mit ihren ringförmigen Querschnittsflächen aneinander, die den jeweiligen Schalenkatalysatorring nach oben und nach unten begrenzen. Die obere Ringfläche des einen Schalenkatalysatorrings haftet (klebt) an der unteren Ringfläche des einen Schalenkatalysatorrings haftet (klebt) an der unteren Ringfläche des anderen Schalenkatalysatorrings. Auf diese Weise entsteht quasi ein Superschalenkatalysatorring, der den gleichen Außendurchmesser A und den gleichen Innendruchmesser I wie die beiden ihn konstituierenden Schalenkatalysatorringe aufweist, dessen Höhe jedoch 2 H beträgt.

Während die Ausbildung von Tandem-Zwillingen bei der Herstellung von ringförmigen Schalenkatalysatoren im wesentlichen unvermeidbar ist, entstehen annellierte Zwillinge bei der Herstellung von ringförmigen Schalenkatalysatoren im wesentlichen (vor allem) dann, wenn H wenigstens > 0,5 A ist.

Insgesamt liegt die Gesamtbildungsmenge M an Schalenkatalysatorringzwillingen bei der Herstellung einer Produktionscharge von ringförmigen Schalenkatalysatoren, bezogen auf das Gesamtgewicht der Produktionscharge, bei ≤ 5 Gew.-%. Meist liegt M, in gleicher Weise bezogen, sogar bei ≤ 4, oder ≤ 3, oder ≤ 2, oder ≤ 1 Gew.-%. Bei sorgfältiger Herstellweise von ringförmigen Schalenkatalysatoren kann M, in gleicher Weise bezogen, sogar ≤ 0,8 Gew.-%, oder ≤ 0,5 Gew.-%, oder ≤ 0,3 Gew.-%, ≤ 0,2 Gew.-%, oder ≤ 0,1 Gew.-% betragen. In der Regel beträgt M jedoch, in gleicher Weise bezogen, > 0, meist ≥ 0,005 und häufig sogar ≥ 0,01 Gew.-%.

Aufgrund der vorgenannten, vergleichsweise geringen, Bildungsmengen von Schalenkatalysatorringzwillingen wurde dem Beisein derselben bei der Verwendung von Produktionschargen ringförmiger Schalenkatalysatoren zur Gestaltung des Katalysatorfestbetts in den Reaktionsrohren von Rohrbündelreaktoren keine erhöhte Bedeutung beigemessen.

Sorgfältigste Untersuchungen der Anmelderin bezüglich der Gestaltung von Katalysatorfestbetten in Reaktionsrohren von Rohrbündelreaktoren unter Mitverwendung von ringförmigen Schalenkatalysatoren haben jedoch zum Ergebnis geführt, dass bei ungünstiger Lage eines Schalenkatalysatorringzwillings im in einem Reaktionsrohr eines Rohrbündelreaktors befindlichen Katalysatorfestbett die Heißpunkttemperatur in diesem Reaktionsrohr bereits durch das Beisein eines einzigen Schalenkatalysatorringzwillings im Katalysatorfestbett spürbar erhöht sein kann.

Eine erhöhte Heißpunkttemperatur ist jedoch gleichbedeutend mit einer beschleunigten Alterung der entsprechenden Katalysatorfestbettbeschickung eines Reaktionsrohres. Um einen solchen beschleunigten Alterungsprozess hinsichtlich der gewünschten Raum-Zeit-Ausbeute an Zielprodukt bei einer in einem Rohrbündelreaktor durchgeführten heterogen katalysierten partiellen Gasphasenoxidation wenigstens temporär zu kompensieren, bedarf es einer beschleunigten Erhöhung von $T_w^{ein}$ des wenigstens einen Wärmeaustauschmittels, was seinerseits eine zusätzliche Beschleunigung des vorgenannten Alterungsprozesses bedingt. Letztendlich resultiert in der Gesamtwirkung eine verminderte Standzeit der Katalysatorfestbettbeschickung des Rohrbündelreaktors, was aus den bereits beschriebenen Gründen unerwünscht ist.

Besonders nachteilig wirkt sich das Beisein eines (oder auch mehrerer) Schalenkatalysatorringzwillings(e) im Katalysatorfestbett eines Thermorohres aus. Der Verlauf der Reaktionstemperatur innerhalb der im Rohrbündelreaktor repräsentativ für alle Arbeitsrohre angeordneten Thermorohre bildet, wie bereits gesagt, die Basis für die Regelung des Gesamtbetriebs eines Rohrbündelreaktors (z. B. die Regelung der Belastung des Katalysatorfestbetts mit Reaktionsgas, die Steuerung der Zusammensetzung des Reaktionsgasgemischs, die Einstellung der jeweiligen $T_w^{ein}$ etc.).

In der Regel richtet sich dabei die Regelung des Gesamtbetriebs aus Sicherheitsgründen an denjenigen Thermorohren aus, deren Betriebsdaten die größte Grenzwertigkeit aufweisen. Sind diese Betriebsdaten infolge eines Beiseins von Schalenkatalysatorringzwillingen im Katalysatorfestbett der entsprechenden Thermorohre für die Betriebsdaten der entsprechenden Arbeitsrohre jedoch nur bedingt repräsentativ, so führt dies normalerweise dazu, dass der gesamte Rohrbündelreaktor nicht in seinem optimalen Betriebszustand betrieben wird.

Vor diesem Hintergrund bestand die Aufgabe der vorliegenden Erfindung darin, ein verbessertes Verfahren zur Beschickung der Reaktionsrohre eines Rohrbündelreaktors mit einem zur Durchführung einer heterogen katalysierten partiellen Gasphasenoxidation einer organischen Ausgangsverbindung geeigneten Katalysatorfestbett, dessen Gestaltung unter Mitverwendung von ringförmigen Schalenkatalysatoren erfolgt, zur Verfügung zu stellen, das die beschriebenen Nachteile der Verfahren des Standes der Technik allenfalls noch in verminderter Form aufweist.

Demgemäß wurde ein Verfahren zum Einbringen einer wenigstens einer Produktionscharge von ringförmigen Schalenkatalysatoren K entnommenen Teilmenge in ein Reaktionsrohr eines Rohrbündelreaktors zum Zweck der Beschickung dieses Reaktionsrohres mit einem zur Durchführung einer heterogen katalysierten partiellen Gasphasenoxidation einer organischen Ausgangsverbindung geeigneten Katalysatorfestbett gefunden, das dadurch gekennzeichnet ist, dass vorab der Entnahme der Teilmenge aus der wenigstens einen Produktionscharge und/oder nach der Entnahme oder vorab der Einbringung der entnommenen Teilmenge in das Reaktionsrohr bei der Herstellung der wenigstens einen Produktionscharge von ringförmigen Schalenkatalysatoren K entstandene Zwillinge von ringförmigen Schalenkatalysatoren K wenigstens teilweise aus der wenigstens einen Produktionscharge und/oder aus der entnommenen Teilmenge entfernt werden.

Erfindungsgemäß vorteilhaft werden vorab der Entnahme der Teilmenge aus der wenigstens einen Produktionscharge von ringförmigen Schalenkatalysatoren K bei der Herstellung derselben entstandene Zwillinge von Schalenkatalysatoren K, bezogen auf den Gesamtgehalt (auf die Gesamtmenge) an in der wenigstens einen Produktionscharge herstellungsbedingt enthaltenen Schalenkatalysatorringzwillingen, zu wenigstens 20 Gew.-%, vorzugsweise zu wenigstens 30 Gew.-%, besonders bevorzugt zu wenigstens 40 Gew.-% und ganz besonders bevorzugt zu wenigstens 50 Gew.-% aus der wenigstens einen Produktionscharge entfernt. Noch besser ist es, wenn beim erfindungsgemäßen Verfahren vorab der Entnahme der Teilmenge aus der wenigstens einen Produktionscharge von ringförmigen Schalenkatalysatoren K, bei der Herstellung derselben entstandenen Zwillinge von Schalenkatalysatoren K, bezogen auf den Gesamtgehalt (auf die Gesamtmenge) an in der wenigstens einen Produktionscharge herstellungsbedingt enthaltenen Schalenkatalysatorringzwillingen, zu wenigstens 60 Gew.-%, oder zu wenigstens 70 Gew.-%, vorzugsweise zu wenigstens 80 Gew.-% oder zu wenigstens 90 Gew.-%, besonders bevorzugt zu wenigstens 95 Gew.-% oder zu wenigstens 98 Gew.-% und ganz besonders bevorzugt zu 100 Gew.-% entfernt werden.

Selbstverständlich können erfindungsgemäß auch erst nach (oder zusätzlich nach) der Entnahme der Teilmenge aus der wenigstens einen Produktionscharge von ringförmigen Schalenkatalysatoren K in dieser entnommenen Teilmenge herstellungsbedingt enthaltene Schalenkatalysatorringzwilling, bezogen auf ihre in der entnommenen Teilmenge enthaltene Gesamtmenge, zu wenigstens 20 Gew.-%, oder zu wenigstens 30 Gew.-%, oder zu wenigstens 40 Gew.-%, oder zu wenigstens 50 Gew.-%, oder zu wenigstens 60 Gew.-%, oder zu wenigstens 70 Gew.-%, oder zu wenigstens 80 Gew.-%, oder zu wenigstens 90 Gew.-%, oder zu wenigstens 95 Gew.-% und ganz besonders bevorzugt zu 100 Gew.-% entfernt (abgetrennt) werden, bevor diese Teilmenge in das Reaktionsrohr eingebracht wird.

Grundsätzlich kann bei heterogen katalysierten partiellen Gasphasenoxidationen das Katalysatorfestbett in jedem einzelnen Reaktionsrohr nur aus der der wenigstens einen Produktionscharge von ringförmigen Schalenkatalysatoren K jeweils entnommenen, im wesentlichen gleich großen, Teilmenge bestehen.

Selbstverständlich kann das Katalysatorfestbett über die Gesamtlänge eines Reaktionsrohres aber auch aus einem homogenisierten Gemisch mehrerer (d. h., wenigstens zwei) voneinander unterscheidbarer Sorten $S_i$ von geometrischen Katalysatorformkörpern oder von geometrischen Katalysatorformkörpern und geometrischen Inertformkörpern bestehen (d. h., ein solches Gemisch kann aus wenigstens zwei voneinander unterscheidbaren Sorten von geometrischen Katalysatorformkörpern, oder aus einer einzigen Sorte von geometrischen Katalysatorformkörpern und aus einer einzigen Sorte von geometrischen Inertformkörpern, oder aus wenigstens zwei Sorten von voneinander unterscheidbaren geometrischen Katalysatorformkörpern und einer einzigen Sorte von geometrischen Inertformkörpern, oder aus wenigsten zwei Sorten von voneinander unterscheidbaren geometrischen Katalysatorformkörpern und wenigstens zwei Sorten von voneinander unterscheidbaren geometrischen Inertformkörpern bestehen).

Unter diesen voneinander verschiedenen Sorten $S_i$ kann sich gegebenenfalls lediglich eine Sorte erfindungsgemäß relevanter ringförmiger Schalenkatalysatoren K befinden. Mögliche Unterscheidungsmerkmale der voneinander verschiedenen Sorten $S_i$ sind die Art der Geometrie, die Art der Aktivmasse, die Art des Trägermaterials etc.

Als Materialien für die geometrischen Inertformkörper (sie dienen dem Zweck, geometrische Katalysatorformkörper in einem Katalysatorfestbett zu verdünnen und auf diese Weise die lokale Wärmeentwicklung im im Reaktionsrohr befindlichen Katalysatorfestbett beim Durchströmen des Reaktionsgasgemischs zu beschränken) kommen prinzipiell die gleichen Materialien in Betracht, die auch für die inerten (z. B. ringförmigen) Trägerformkörper zur Herstellung von Schalenkatalysatoren verwendet werden können und in den Ablauf der Gasphasenpartialoxidation im wesentlichen nicht eingreifen.

Letzteres heißt hier in der Regel, dass wenn das Reaktionsgasgemisch unter denselben Reaktionsbedingungen durch ein nur mit inerten Trägerformkörpern (inerten Verdünnungsformkörpern) beschicktes Reaktionsrohr geführt wird, der Umsatz der partiell zu oxidierenden organischen Ausgangsverbindung ≤ 5 mol-%, meist ≤ 2 mol-% beträgt.

Als solche Inertmaterialien für geometrische Trägerformkörper bzw. inerte Verdünnungsformkörper kommen für zahlreiche heterogen katalysierte partielle Gasphasenoxidationen z. B. poröse oder unporöse Aluminiumoxide, Siliciumdioxid (bzw. allgemein Siliciumoxid), Thoriumdioxid, Zirkonoxid, Siliciumcarbid oder Silikate wie Magnesium- oder Aluminiumsilikat (z. B. Steatit des Typs C 220 der Fa. CeramTec), aber auch Metalle wie z. B. Edelstahl oder Aluminium (vgl. z. B. US 2006/0205978) in Betracht.

Grundsätzlich kommen alle inerten Trägerformkörper auch als geometrische Inertformkörper zur Verdünnung von geometrischen Katalysatorformkörpern in einem Katalysatorfestbett in Betracht.

Durch eine solche Verdünnung kann die volumenspezifische Aktivität eines Katalysatorfestbetts, wie bereits gesagt, auf den Bedarf der jeweiligen heterogen katalysierten partiellen Gasphasenoxidation spezifisch eingestellt werden.

Vorzugsweise weisen geometrische Inertformkörper und geometrische Katalysatorformkörper in einem dem Vorgenannten entsprechend homogenisierten Gemisch die gleiche, oder wenigstens eine einander ähnliche Geometrie auf.

Der Wortlaut "homogenisiertes Gemisch" meint dabei, dass Maßnahmen ergriffen worden sind, um die voneinander verschiedenen Sorten geometrischer Formkörper (bzw. die verschiedenen Längstausdehnungen innerhalb einer Sorte) miteinander homogen zu vermengen. Im Idealfall erreicht die homogene Vermengung entlang des gesamten Längsabschnitts den statistischen Durchschnitt und dies auch bezüglich der jeweiligen individuellen Sorte.

Vielfach besteht eine Reaktionsrohrbeschickung (eine Reaktionsrohrbefüllung) mit einem Katalysatorfestbett aber auch aus mehreren voneinander unterscheidbar übereinander (hintereinander) angebrachten Längsabschnitten (Katalysatorfestbett(längs)abschnitten, Katalysatorschüttungsabschnitten). Jeder einzelne Längsabschnitt kann dabei über seine Länge einheitlich so gestaltet werden, wie es für ein über seine gesamte Reaktionsrohrlänge einheitlich beschicktes Reaktionsrohr bereits ausgeführt wurde. Beim Übergang von einem in sich einheitlichen Schüttungsabschnitt zum nächsten in sich einheitlichen Schüttungsabschnitt ändert sich die Zusammenstellung (Zusammensetzung) der Schüttung normalerweise abrupt. Es entstehen so längs eines individuellen Reaktionsrohres Katalysatorfestbettschüttungen, die eine heterogene Struktur aufweisen. Man spricht auch von einer strukturierten Befüllung (bzw. Schüttung) der Reaktionsrohre.

Am Anfang (in Strömungsrichtung des das Reaktionsrohr durchströmenden Reaktionsgases geblickt) und/oder am Ende des Reaktionsrohres wird das Katalysatorfestbett häufig durch eine alleinige Schüttung aus geometrischen Inertformkörpern abgeschlossen. Derartige alleinige Inertschüttungen werden üblicherweise nicht dem Katalysatorfestbett zugerechnet.

Anwendungstechnisch zweckmäßig wird man im Fall einer strukturierten Befüllung der Reaktionsrohre den Inhalt eines in sich einheitlichen Schüttungsabschnitts der Lehre der DE-A 10 2004 023249 folgend in homogenisierter Form vorab erzeugen und als eine solche Schüttungsabschnittsportion verpacken. Sind ringförmige Schalenkatalysatoren K Bestandteile einer solchen vorab verpackten Schüttungsabschnittsportion, so erfolgt das erfindungsgemäße Einbringen derselben in das Reaktionsrohr als Bestandteil der entsprechenden Schüttungsabschnittsportion. D. h., zur Vorabpräparation der Schüttungsabschnittsportion wird der wenigstens einen Produktionscharge von ringförmigen Schalenkatalysatoren K (erfindungsgemäß bevorzugt), nachdem im Rahmen ihrer Herstellung entstandene und dadurch in ihr enthaltene Schalenkatalysatorringzwillinge in erfindungsgemäßer Weise wenigstens teilweise aus ihr entfernt worden sind, die erforderliche Teilmenge entnommen, mit den anderen geometrischen Formkörpern der Schüttungsabschnittsportion homogenisiert, das dabei resultierende homogenisierte Gemisch geometrischer Formkörper in die Verpackung eingebracht und die so erzeugte Packung im Rahmen der Beschickung der Reaktionsrohre mit dem gewünschten Katalysatorfestbett ins Reaktionsrohr entleert. D. h., das erfindungsgemäße Einbringen einer wenigstens einer Produktionscharge von ringförmigen Schalenkatalysatoren K entnommenen Teilmenge in ein Reaktionsrohr eines Rohrbündelreaktors muss nicht in notwendiger Weise separat, sondern kann auch im homogenisierten Gemisch mit anderen geometrischen Formkörpern erfolgen (selbstverständlich kann die Zwillingsabtrennung auch erst nach der Teilmengenentnahme aus der wenigstens einen Produktionscharge erfolgen).

In der Regel wird die Befüllung eines Reaktionsrohres mit einem strukturierten Katalysatorfestbett so gestaltet, dass die volumenspezifische Aktivität des Katalysatorfestbetts in Strömungsrichtung des Katalysatorfestbetts zunimmt.

Dies ist z. B. in einfacher Weise dadurch realisierbar, dass das Katalysatorfestbett aus voneinander verschiedenen Längsabschnitten besteht, die sich lediglich dadurch voneinander unterscheiden, dass eine Sorte von ringförmigen Schalenkatalysatoren K mit unterschiedlichen Mengenanteilen nur einer Sorte von Inertformkörpern verdünnt ist. In Strömungsrichtung des Reaktionsgases nimmt der Verdünnungsgrad mit Inertformkörpern ab und häufig wird der in Strömungsrichtung letzte Längsabschnitt des Katalysatorfestbetts nur aus ringförmigen Schalenkatalysatoren K bestehen.

Eine in Strömungsrichtung des Reaktionsgases zunehmende volumenspezifische Aktivität des Katalysatorfestbetts kann jedoch auch dadurch verwirklicht werden, dass zwar die einzelnen Längsabschnitte des Katalysatorfestbetts jeweils nur aus einer Sorte an ringförmigem Schalenkatalysator bestehen, wobei die Ringgeometrien der von einander verschiedenen Schalenkatalysatoren üblicherweise im wesentlichen die gleichen sind, die Aktivmassen sich aufgrund einer unterschiedlichen Elementzusammensetzung jedoch voneinander unterscheiden, mit der Maßgabe, dass die katalytische Aktivität der jeweils verwendeten Aktivmasse in Strömungsrichtung des Reaktionsgasgemischs zunimmt. Die volumenspezifische Aktivität eines in sich einheitlichen Längsabschnitts einer Katalysatorfestbettbeschickung eines Reaktionsrohres ist normalerweise dann erhöht, wenn bei durchgehender Beschickung des Reaktionsrohres wie im entsprechenden Längsabschnitt des Reaktionsrohres unter ansonsten identischen Reaktionsbedingungen (d. h., identische Zusammensetzung des Reaktionsgasgemischs, identische Belastung der Katalysatorfestbettbeschickung mit Reaktionsgasgemisch sowie identische Eintrittstemperatur des Wärmeträgers und identische Strömungsbedingungen des Wärmeträgers) ein erhöhter Umsatz der partiell zu oxidierenden organischen Ausgangsverbindung resultiert.

Beispiele für strukturierte Befüllungen von Reaktionsrohren zum Zweck der Durchführung von heterogen katalysierten partiellen Gasphasenoxidationen mit in Strömungsrichtung des Reaktionsgasgemischs zunehmender volumenspezifischer Aktivität unter Mitverwendung von ringförmigen Schalenkatalysatoren offenbaren z. B. die Schriften EP-A 173 4030, DE-A 19823262, DE-A 19823275, Deutsche Anmeldung mit dem Aktenzeichen 102007010422.9, EP-A 1734030 und Deutsche Anmeldung mit dem Aktenzeichen 102007019597.6.

Das erfindungsgemäße Verfahren ist dann von besonderer Relevanz, wenn das in die Reaktionsrohre eingebrachte Katalysatorfestbett zu wenigstens 20 %, bzw. zu wenigstens 30 %, oder zu wenigstens 40 %, bzw. zu wenigstens 50 %, oder zu wenigstens 60 %, bzw. zu wenigstens 70 %, oder zu wenigstens 80 %, bzw. zu wenigstens 90 %, oder zu wenigstens 95 %, bzw. zu 100 % seines Gewichtes aus ringförmigen Schalenkatalysatoren besteht.

Erfindungsgemäß bevorzugt werden wenigstens 20 Gew.-%, vorzugsweise wenigstens 40 Gew.-%, besonders bevorzugt wenigstens 60 Gew.-%, ganz besonders bevorzugt wenigstens 80 Gew.-% und am Besten die Gesamtmenge der in einem Reaktionsrohr befindlichen ringförmigen Schalenkatalysatoren nach der erfindungsgemäßen Verfahrensweise in das Reaktionsrohr eingebracht.

Erfindungsgemäß vorteilhaft werden insbesondere diejenigen ringförmigen Schalenkatalysatoren eines in einem Reaktionsrohr befindlichen Katalysatorfestbetts mit der erfindungsgemäßen Verfahrensweise in das Reaktionsrohr eingebracht, die sich, in Strömungsrichtung des Reaktionsgasgemischs blickend, auf den ersten 80 %, bzw. auf den ersten 60 %, oder auf den ersten 40 %, bzw. auf den ersten 20 % der Gesamtkatalysatorfestbett(schüttungs)länge befinden.

Grundsätzlich gelten alle in dieser Schrift gemachten Aussagen über heterogen katalysierte partielle Gasphasenoxidationen organischer Ausgangsverbindung und deren Durchführung im in den Reaktionsrohren eines Rohrbündelreaktors befindlichen Katalysatorfestbett vor allem dann, wenn die Reaktionsrohre in erfindungsgemäßer Weise mit dem Katalysatorfestbett beschickt worden sind.

Der Wasserdampfgehalt des Reaktionsgaseingangsgemisch kann bei diesen heterogen katalysierten partiellen Gasphasenoxidationen grundsätzlich 0 (verschwindend) sein.

Normalerweise wird der Wasserdampfgehalt des Reaktionsgaseingangsgemischs bei diesen heterogen katalysierten partiellen Gasphasenoxidationen normalerweise jedoch > 0 Vol.-% betragen.

Häufig wird der Wasserdampfgehalt des Reaktionsgaseingangsgemisch $\geq$ 0,1 bis 60 Vol.-%, oder $\geq$ 0,2 bis 50 Vol.-% oder $\geq$ 0,3 bis 40 Vol.-%, oder $\geq$ 0,4 bis 30 Vol.-%, oder $\geq$ 0,5 bis 25 Vol.-%, oder $\geq$ 0,75 bis 20 Vol.-%, oder $\geq$ 1 bis 15 Vol.-%, oder $\geq$ 2 bis 10 Vol.-% betragen. Aufgrund seiner vergleichsweise erhöhten spezifischen Wärmekapazität ist Wasserdampf in der Regel ein hervorragendes inertes Verdünnungsgas für heterogen katalysierte partielle Gasphasenoxidationen organischer Ausgangsverbindungen und wirkt sich vielfach förderlich auf die Katalysatoraktivität aus.

Als Quelle für den für eine heterogen katalysierte partielle Gasphasenoxidation erforderlichen molekularen Sauerstoff im Reaktionsgaseingangsgemisch kommen sowohl Luft, reiner molekularer Sauerstoff, an molekularem Sauerstoff entreicherte Luft oder sonstige Gemische aus Inertgas und molekularem Sauerstoff in Betracht.

Der Gehalt des Reaktionsgaseingangsgemischs an der heterogen katalysiert partiell zu oxidierenden organischen Ausgangsverbindung kann bei heterogen katalysierten partiellen Gasphasenoxidationen, für die das erfindungsgemäße Verfahren relevant ist (das sind insbesondere alle in dieser Schrift diskutierten heterogen katalysierten partiellen Gasphasenoxidationen), bis zu 50 Vol.-% oder mehr betragen.

Häufig wird dieser Gehalt ≥ 2 bis 20 Vol.-%, oder ≥ 4 bis 12 Vol.-% betragen. Enthält das Reaktionsgaseingangsgemisch den molekularen Sauerstoff in einer bezogen auf die angestrebte Partialoxidation unterstöchiometrischen Menge, kann die im Reaktionsgaseintrittsgemisch enthaltene Überschussmenge an der partiell zu oxidierenden organischen Ausgangsverbindung grundsätzlich als inertes Verdünnungsgas fungieren. Enthält das Reaktionsgaseingangsgemisch molekularen Sauerstoff in auf die Partialoxidation bezogen überstöchiometrischer Menge, wird selbige dabei anwendungstechnisch zweckmäßig häufig so gewählt, dass die Zusammensetzung des Reaktionsgaseingangsgemischs außerhalb des explosionsfähigen Zusammensetzungsbereichs liegt.

Selbstverständlich kann die Zusammensetzung des Reaktionsgaseingangsgemischs aber auch im explosionsfähigen Zusammensetzungsbereich liegen, wie es z. B. im Fall der Herstellung von Phthalsäureanhydrid aus o-Xylol und/oder Naphthalin normalerweise gegeben ist.

Aus Gründen einer möglichst langen Katalysatorstandzeit wird man den Anteil des molekularen Sauerstoff im Reaktionsgaseingangsgemisch einer heterogen katalysierten partiellen Gasphasenoxidation, für die das erfindungsgemäße Verfahren relevant ist, in der Regel vorzugsweise so wählen, dass das Produktgasgemisch der Gasphasenpartialoxidation noch überschüssigen molekularen Sauerstoff (z. B. bis zu 3 Vol.-%) enthält.

Der Volumenstrom des Temperiermediums (des wenigstens einen Wärmeaustauschmittels (vorzugsweise eines flüssigen)) im Reaktionsrohrumgebungsraum wird bei erfindungsgemäß relevanten Gasphasenpartialoxidationen üblicherweise so bemessen, dass der Temperaturanstieg (bedingt durch die Exothermie der Partialoxidation) des (vorzugsweise flüssigen) wenigstens einen Wärmeaustauschmittels von seiner Eintrittsstelle in den Rohrbündelreaktor bis zu seiner Austrittsstelle aus dem Rohrbündelreaktor ≥ 0 bis 15 °C, bzw. ≥ 0 bis 10 °C, häufig ≥ 2 bis 8 °C, bevorzugt ≥ 3 bis 6 °C beträgt.

Die Belastung des Katalysatorfestbetts mit der partiell zu oxidierenden organischen Ausgangsverbindung wird bei einer erfindungsgemäß relevanten Gasphasenpartialoxidation in der Regel ≥ 50 Nl/l•h, meist ≥ 75 Nl/l•h, vielfach ≥ 100 Nl/l•h betragen. Meist wird diese Belastung jedoch bei ≤ 600 Nl/l•h liegen.

Die Belastung des Katalysatorfestbetts mit Reaktionsgaseingangsgemisch wird bei einer erfindungsgemäß relevanten Gasphasenpartialoxidation häufig ≥ 1500 Nl/l•h, oder ≥ 2000 Nl/l•h, oder ≥ 2500 Nl/l•h, oder ≥ 3000 Nl/l•h, oder ≥ 4000 Nl/l•h betragen. In der Regel liegt vorgenannte Belastung bei solchen heterogen katalysierten partiellen Gasphasenoxidationen jedoch bei Werten von ≤ 6000 Nl/l•h, bzw. ≤ 5000 Nl/l•h. Der Umsatz der partiell zu oxidierenden organischen Ausgangsverbindung wird bei erfindungsgemäß relevanten Gasphasenpartialoxidationen in typischer Weise ≥ 50 mol-%, häufig ≥ 70 mol-%, vielfach ≥ 80 mol-% und oft ≥ 90 mol-% betragen (bezogen auf den einmaligen Durchgang des Reaktionsgasgemischs durch das Katalysatorfestbett). Die Selektivität der Zielproduktbildung wird dabei in typischer Weise ≥ 70 mol-%, häufig ≥ 80 mol-% und vielfach ≥ 90 mol-% betragen.

Im übrigen werden die Rahmenbedingungen einer erfindungsgemäß relevanten heterogen katalysierten partiellen Gasphasenoxidation anwendungstechnisch zweckmäßig insgesamt normalerweise so gewählt, dass der Temperaturunterschied zwischen der Heißpunkttemperatur des Reaktionsgasgemischs in den einzelnen Reaktionszonen (Temperaturzonen) des Rohrbündelreaktors und der jeweiligen zugehörigen $T_w^{ein}$ der Temperaturzone auch im Langzeitbetrieb in der Regel 100°C nicht überschreitet. Bei Partialoxidationen mit nicht so ausgeprägter Wärmetönung beträgt dieser Temperaturunterschied auch im Langzeitbetrieb häufig ≤ 80°C oder ≤ 70°C, vielfach liegt er bei 20 bis 70°C bzw. bis 50°C, vorzugsweise ist dieser Temperaturunterschied auch im Langzeitbetrieb gering.

Außerdem werden die vorgenannten Rahmenbedingungen üblicherweise so gewählt, dass die "peak-to-salt temperatur sensitivity (vgl. Definition in der EP-A 1106598) insbesondere auch im Langzeitbetrieb ≤ 9 °C, aber ≤ 7 °C, oder ≤ 5 °C, oder ≤ 3 °C beträgt. Damit wird unter anderem dem Sachverhalt Rechnung getragen, dass die Temperatur des wenigstens einen Wärmeaustauschmittels über den Querschnitt des Rohrbündelreaktors betrachtet in der Regel nicht völlig homogen (einheitlich) ist, sondern normalerweise einen geringen Gradienten aufweist.

Der Außendurchmesser A der ringförmigen Trägerformkörper von erfindungsgemäß in ein Reaktionssrohr einzubringenden Schalenkatalysatoren K beträgt in typischer Weise 4 bis 10 mm, die zugehörige Höhe (Länge) H 2 bis 10 mm und ihre Wanddicke liegt in der Regel bei 1 bis 4 mm.

Vorzugsweise beträgt der Außendurchmesser A solcher ringförmigen Trägerformkörper 4 bis 8 mm, die Wanddicke 1 bis 2 mm und die Höhe H 3 bis 7 mm.

Besonders häufig angewandte Geometrien von ringförmigen Trägerformkörpern von erfindungsgemäß einzubringenden Schalenkatalysatoren K sind die Ringgeometrien (A (Außendurchmesser) x I (Innendurchmesser) x H (Höhe)) 8 mm x 5 mm x 6 mm, 7 mm x 4 mm x 7 mm, 7 mm x 4 mm x 4 mm sowie 7 mm x 4 mm x 3 mm.

Die Dicke der auf den ringförmigen Trägerformkörper aufgebrachten Aktivmassenschale liegt in der Regel bei 10 bis 3000 bzw. bis 1000 $\mu$m, vorzugsweise bei 10 bis 500 $\mu$m, häufig bei 100 bis 500 $\mu$m und vielfach bei 200 bis 300 $\mu$m. Bei der auf die ringförmigen Trägerformkörper aufgebrachten katalytischen Aktivmasse handelt es sich bei Schalenkatalysatoren K in der Regel um wenigstens ein Multielementoxid (häufig um wenigstens ein Multimetalloxid) oder um Massen, die wenigstens ein Multielementoxid (z. B. Multimetalloxid) enthalten.

Grundsätzlich meint der Begriff Multielementoxid in dieser Schrift, dass die katalytisch aktive Oxidmasse neben Sauerstoff noch wenigstens zwei weitere voneinander verschiedene Elemente enthält. Besonders häufig kommen als katalytisch aktive Multielementoxidmassen solche zur Anwendung, die wenigstens zwei metallische, insbesondere wenigstens zwei übergangsmetallische Elemente aufweisen. In diesem Fall spricht man von Multimetalloxidmassen. Im Regefall sind katalytisch aktive Multielementoxidmassen keine einfachen physikalischen Gemische von Oxiden ihrer elementaren Konstituenten, sondern heterogene Gemisch von komplexen Polyverbindungen dieser Elemente. Grundsätzlich kommen als katalytisch aktive Multielementoxidmassen aber auch einfache physikalische Gemisch (z. B. Agglomerate von feinteiligen Elementoxiden) von Oxiden ihrer elementaren Konstituenten in Betracht (z. B. bei den ringförmigen Schalenkatalysatoren K zur Herstellung von Phthalsäureanhydrid aus o-Xylol und/oder Naphthalin), weshalb in dieser Schrift der Oberbegriff "Multielementoxidmassen" solche Gemische (Agglomerate) subsummieren soll.

In einer Vielzahl von Fällen ist das wenigstens eine katalytisch aktive Multielementoxid ein solches, das

    a) die Elemente Mo, Fe und Bi, oder

    b) die Elemente Mo und V, oder

    c) das Element V sowie zusätzlich P und/oder Ti

enthält. Zusätzlich kommen als erfindungsgemäß in ein Reaktionsrohr einzubringende Schalenkatalysatoren solche in Betracht, die als Aktivmasse elementares Silber auf einem oxidischen ringförmigen Trägerformkörper enthalten.

Eine Abtrennung von bei der Herstellung der wenigstens einen Produktioncharge von ringförmigen Schalenkatalysatoren K entstandenen Zwillingen von ringförmigen Schalenkatalysatoren K aus der wenigstens einen Produktioncharge kann in einfachster Weise manuell, d. h., durch händisches Auslesen erfolgen. Alternativ kann die Abtrennung auch durch Windsichten vorgenommen werden, bei dem das unterschiedliche Gewicht von Schalenkatalysatoren K und ihren Zwillingen ausgenutzt wird.

Beispielsweise kann man die Gesamt- oder eine Teilmenge der wenigstens einen Produktioncharge durch eine Schleuse führen, die nur dem Zutritt eines einzelnen Schalenkatalysatorringes K öffnet (die Detektion kann z. B. mittels optischer Methode erfolgen). Wird die Schleuse in der Folgewirkung durch einen Zwilling blockiert, wird dieser durch einen entsprechend ausgerichteten Gasstrom (z. B. einen Luftstrom) weggeblasen (d. h., wird der Schleuseneingang freigeblasen). Hinter der Schleuse kann dann zum Einbringen in ein Produktionsrohr ringförmiger Schalenkatalysator K entnommen werden.

Anwendungstechnisch besonders vorteilhaft wird man eine Abtrennung von bei der Herstellung der wenigstens einen Produktioncharge von ringförmigen Schalenkatalysatoren K entstandenen Zwillingen von ringförmigen Schalenkatalysatoren K jedoch durch ein Verfahren der Siebung vornehmen. Dabei verbleiben als Siebrückstand (auch als "Überkorn" bezeichnet) normalerweise im wesentlichen die Zwillinge (sowie die gegebenenfalls im Rahmen der Herstellung der ringförmigen Schalenkatalysatoren K gebildeten sonstigen Mehrlinge von ringförmigen Schalenkatalysatoren K), während der Siebdurchgang (auch als "Unterkorn" bezeichnet) üblicherweise im wesentlichen die ringförmigen Schalenkatalysatoren K umfasst.

Gilt für die ringförmige Geometrie A x I x H des Schalenkatalysators K (A = Außendurchmesser, I = Innendurchmesser, H = Höhe) die Beziehung $H \leq 0,5 \cdot A$, so ist die Ausbildung von annellierten Schalenkatalysatorringzwillingen bei der Herstellung der ringförmigen Schalenkatalysatoren K im Vergleich zur Ausbildung von Tandem-Schalenkatalysatorringzwillingen gemäß den Untersuchungen der Anmelderin mit den meisten üblichen Bindemitteln in der Regel mengenmäßig vernachlässigbar.

Vor diesem Hintergrund empfiehlt sich zur erfindungsgemäßen Entfernung von Zwillingen aus der wenigstens einen Produktioncharge von vorgenannten ringförmigen Schalenkatalystoren K (oder aus der dieser entnommenen Teilmenge) ein Verfahren der Siebung mit Hilfe eines Siebes, das Sieböffnungen $O_1$ aufweist, in deren geschlossene Umrisslinie ein Rechteck R mit den Seitenlängen L und C zwar unter der Maßgabe $M_1$,

$$L > A \geq 2H > C > H,$$

aber nicht mehr unter der Maßgabe $M_1^*$,

$$L > C \geq 2\,H,$$

einbeschrieben werden kann.

Vorzugsweise empfiehlt sich im Fall der vorgenannten ringförmigen Schalenkatalysatoren K ein Verfahren der Siebung mit Hilfe eines Siebes, das Sieböffnungen $O_2$ aufweist, in deren geschlossene Umrisslinie ein Rechteck R mit den Seitenlängen L und C zwar unter der Maßgabe $M_2$,

$$L > A \geq 2\,H > 1{,}75\,H \geq C \geq 1{,}25\,H,$$

Aber nicht mehr unter Maßgabe $M_2{}^*$,

$$L > C > 1{,}75\,H,$$

einbeschrieben werden kann.

Sowohl im Fall der Sieböffnungen $O_1$ als auch im Fall der Sieböffnungen $O_2$ ist es erfindungsgemäß günstig, wenn $L \geq 1{,}05 \cdot A$, besser $\geq 1{,}1 \cdot A$, vorzugsweise $\geq 1{,}25 \cdot A$, besonders bevorzugt $\geq 1{,}5 \cdot A$ und ganz besonders bevorzugt $\geq 1{,}75 \cdot A$ beträgt.

Grundsätzlich kann sowohl im Fall der Sieböffnungen $O_1$ als auch im Fall der Sieböffnungen $O_2$ $L \geq 2\,A$, oder $\geq 2{,}5 \cdot A$ betragen. In der Regel wird L jedoch sowohl im Fall der Sieböffnungen $O_1$ als auch im Fall der Sieböffnungen $O_2 \leq 20 \cdot A$, vielfach $\leq 15 \cdot A$, häufig $\leq 10 \cdot A$ und oft $\leq 5 \cdot A$ betragen. Diese Längenbeschränkung ist jedoch häufig eher durch Sekundärmerkmale wie z. B. eine ausgezeichnete mechanische Stabilität des Siebes, als durch die angestrebte Siebwirkung bedingt.

Für den Fall, das in den vorgenannten Fällen zusätzlich eine siebtechnische Abtrennung von annelierten Zwillingen angestrebt wird, sollte für die Maßgabe $M_1$,

$$2\,A > L > A \geq 2\,H > C > H,$$

vorzugsweise sogar

$$1{,}9\,A > L > A \geq 2\,H > C > H,$$

und für die Maßgabe
$M_2$ in entsprechender Weise

$$2\,A > L > A \geq 2\,H > 1{,}75\,H \geq C \geq 1{,}25\,H,$$

und vorzugsweise sogar

$$1{,}9\,A > L > A \geq 2\,H > 1{,}75\,H \geq C \geq 1{,}25\,H$$

erfüllt sein.

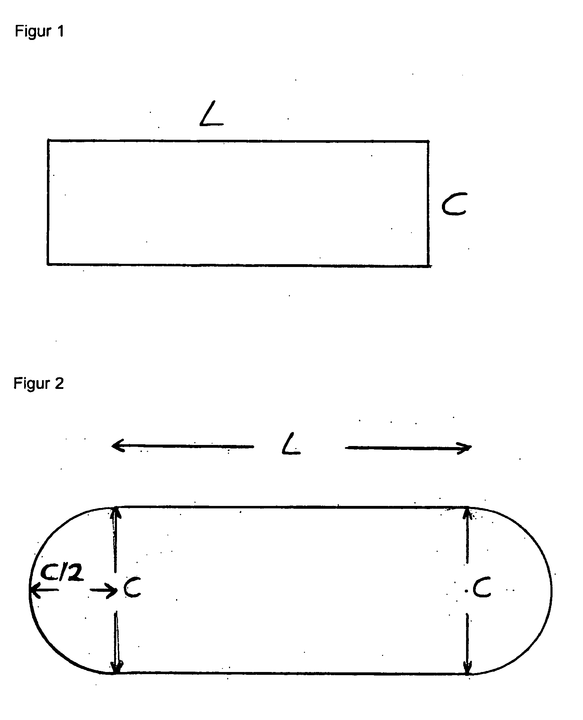

Anwendungstechnisch zweckmäßig handelt es sich sowohl bei der geschlossenen Umrißlinie der Sieböffnungen $O_1$ als auch bei der geschlossenen Umrißlinie der Sieböffnungen $O_2$ um ein Rechteck mit den Seitenlängen L und C (im vereinfachten Sprachgebrauch wird in dieser Schrift die geometrische Form der geschlossenen Umrisslinie einer Sieböffnung häufig auch als "geometrische Form der Sieböffnung" bezeichnet), wie es die Figur 1 zeigt.

Selbstverständlich kann es sich sowohl bei einer Sieböffnung $O_1$ als auch bei einer Sieböffnung $O_2$ aber auch um ein Langloch handeln, wie es beispielhaft die Figur 2 zeigt.

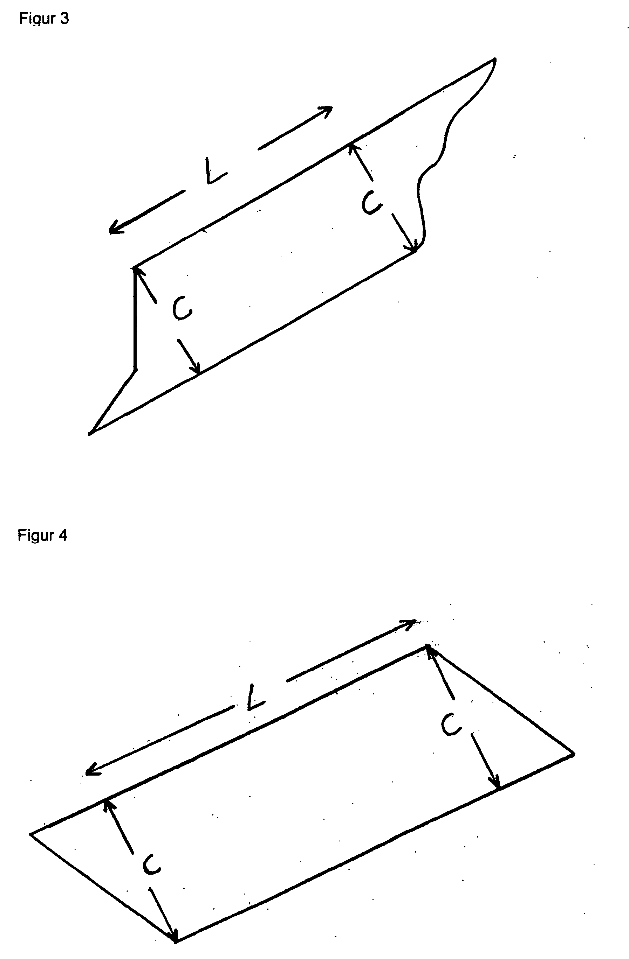

Die Geometrie eines solchen Langlochs leitet sich von derjenigen des relevanten Rechtecks mit den Seitenlängen L und C in einfacher Weise dadurch ab, dass die Rechteckseiten mit der Länge C jeweils durch einen Halbkreis mit dem Durchmesser C (der Lochweite) ersetzt sind, wobei die Halbkreiswölbung aus der Rechteckfläche herauszeigt. Eine vergleichsweise allgemeine Form einer möglichen im vorbeschriebenen Sinn erfindungsgemäß geeigneten Sieböffnung (bzw. deren Umrisslinie) zeigt beispielhaft die Figur 3. Natürlich ist als im vorbeschriebenen Sinn erfindungsgemäß geeignete Sieböffnung (bzw. deren Umrisslinie (beide Ausdrucksweisen werden in dieser Schrift, wie bereits gesagt, äquivalent verwendet)) auch ein Parallelogramm möglich, wie es beispielhaft die Figur 4 ausweist. Ferner kommt als Umrisslinie einer im vorbeschriebenen Sinn erfindungsgemäß geeigneten Sieböffnung auch eine solche in Betracht, die sich von einer rechteckigen Umrisslinie dadurch ableitet, dass alle oder wenigstens eine Teilmenge der Ecken des Rechtecks abgerundet worden sind.

Gelten für die ringförmige Geometrie A x I x H des ringförmigen Schalenkatalysators K die Beziehungen $A \geq H > 0{,}5 \cdot A$, so wird die Ausbildung von annelierten Schalenkatalysatorringzwillingen bei der Herstellung von ringförmigen Schalenkatalysatoren K gemäß den Untersuchungen der Anmelderin mit zunehmendem Verhältnis H/A (in Abhängigkeit vom verwendeten Bindemittel) zunehmend bedeutend.

Vor diesem Hintergrund empfiehlt sich zur erfindungsgemäßen Entfernung von Zwillingen aus der wenigstens einen Produktionscharge (oder aus der dieser entnommenen Teilmenge) von solchen (vorgenannten) ringförmigen Schalenkatalysatoren K ein Verfahren der Siebung mit Hilfe eines Siebes, das Sieböffnungen $O_3$ aufweist, in deren geschlossene Umrißline ein Rechteck R mit den Seitenlängen L und C zwar unter der Maßgabe M3,

$$L > A < 2\,H > C > H,$$

aber nicht mehr unter Maßgabe $M_3{}^*$,

$$L \geq C \geq 2\,H,$$

einbeschrieben werden kann.

Vorzugsweise empfiehlt sich im Fall der vorgenannten ringförmigen Schalenkatalysatoren K ein Verfahren der Siebung mit Hilfe eines Siebes, das Sieböffnungen $O_4$ aufweist, in deren geschlossene Umrisslinie ein Rechteck R mit den Seitenlängen L und C zwar unter der Maßgabe $M_4$,

$$2\,A > L > A < 2\,H > C > H,$$

aber nicht mehr unter der Maßgabe $M_4{}^*$,

$$L \geq C \geq 2\,H,$$

einbeschrieben werden kann.

Besonders bevorzugt empfiehlt sich im Fall der vorgenannten ringförmigen Schalenkatalysatoren K ein Verfahren der Siebung mit Hilfe eines Siebes, das Sieböffnungen $O_5$ aufweist, in deren geschlossene Umrisslinie ein Rechteck R mit den Seitenlängen L und C zwar unter der Maßgabe $M_5$,

$$2\,H > L > A < 2\,H > C > H,$$

aber nicht mehr unter Maßgabe $M_5{}^*$,

$$L \geq C \geq 2\,H$$

einbeschrieben werden kann.

Erfindungsgemäß bevorzugt handelt es sich bei dem in die Sieböffnungen $O_3$, $O_4$ oder $O_5$ einzubeschreibenden Rechtecken R um deren Sonderform eines Quadrates mit $L = C$. Letzteres erweist sich insbesondere für den Siebdurchsatz als förderlich.

Anwendungstechnisch zweckmäßig handelt es sich auch bei den geschlossenen Umrisslinien der Sieböffnungen $O_3$, $O_4$ bzw. $O_5$ jeweils um ein Rechteck mit den Seitenlängen L und C (vorzugsweise um dessen Sonderform eines Quadrates mit $L = C$).

Selbstverständlich kann es sich aber auch bei einer Sieböffnung $O_3$, oder $O_4$ bzw. $O_5$ um ein Langloch handeln (das sich hier auch von einem Quadrat ableiten kann). Natürlich ist als solche Umrisslinie auch ein Parallelogramm möglich oder ein Rechteck, dessen Ecken alle oder wenigstens eine Teilmenge von ihnen abgerundet worden sind.

Grundsätzlich kann ein erfindungsgemäß zu verwendendes Sieb z. B. mehrere voneinander verschiedene Sorten von erfindungsgemäß möglichen Sieböffnungen aufweisen. Erfindungsgemäß vorteilhaft wird ein bei einem erfindungsgemäßen Verfahren eingesetztes Sieb jedoch nicht mehr als drei, und in der Regel nicht mehr als zwei voneinander verschiedene, das erfindungsgemäße Anforderungsprofil erfüllende, Sorten von Sieböffnungen aufweisen. Ganz besonders vorteilhaft wird ein erfindungsgemäß zu verwendendes Sieb jedoch nur eine Sorte von erfindungsgemäßen Sieböffnungen aufweisen.

Der Begriff "Sieb" wird in dieser Schrift synonym mit dem Begriff "Siebboden" verwendet. Im übrigen wird der Begriff "Sieb" oder "Siebboden" in dieser Schrift im Sinne der in der in der EP-A 1 726 358 in Spalte 5, Zeilen 48 bis 57 gegebenen Begriffsdefinition verwendet.

D. h., der Siebboden kann beispielsweise als Gitter bzw. Rost, als Loch- bzw. Spaltblech (d. h., als Blech mit gestanzten, gelaserten, wassergeschnittenen oder gefrästen Sieböffnungen) oder als Siebgewebe (es besteht aus miteinander verwobenen Drähten, wobei die Drähte rund oder profiliert sein können) ausgebildet sind. Grundsätzlich kommt für eine erfindungsgemäßes Siebverfahren aber auch jede andere in "Aufbereitungs-Technik-Nr. 11/1960, S. 457 bis 473 oder in Chem.-Ing.-Techn. 56 (1984) Nr. 12, Seite 897 bis 907 aufgeführte Siebbodenvariante in Betracht. Selbstverständlich können für ein erfindungsgemäßes Siebverfahren aber auch alle in "Sieben und Siebmaschinen, Wiley-VCH GmbH & Co. KGaA, Paul Schmidt et al (2003)" aufgeführten Siebböden verwendet werden.

Gitter bzw. Roste sowie Siebgewebe (beide gewährleisten besonders hohe spezifische Siebleistungen in $kg/m^{3 \cdot} h$ bei hohem Wirkungsgrad) eignen sich insbesondere im Fall von nur eine erfindungsgemäße Sorte einer rechteckigen Sieböffnung aufweisenden Siebböden. Eine beispielhafte veranschaulichende Abbildung eines solchen Siebgewebes zeigt die Figur 5 dieser Schrift.

Eine beispielhafte veranschaulichende Abbildung eines solchen Gitters bzw. Rostes zeigt die Figur 6 dieser Schrift.

Beliebige erfindungsgemäß geeignete Sieböffnungen (bzw. Umrisslinien von Sieböffnungen) können auf einfache Weise in Loch- bzw. Spaltblechen verwirklicht werden. Erfindungsgemäß vorteilhafte Loch- bzw. Spaltbleche sind jedoch insbesondere solche, die lediglich eine Sorte einer rechteckigen (bzw. quadratischen) bzw. einer Langlochform aufweisenden Sieböffnung (bzw. deren Umrisslinie) aufweisen.

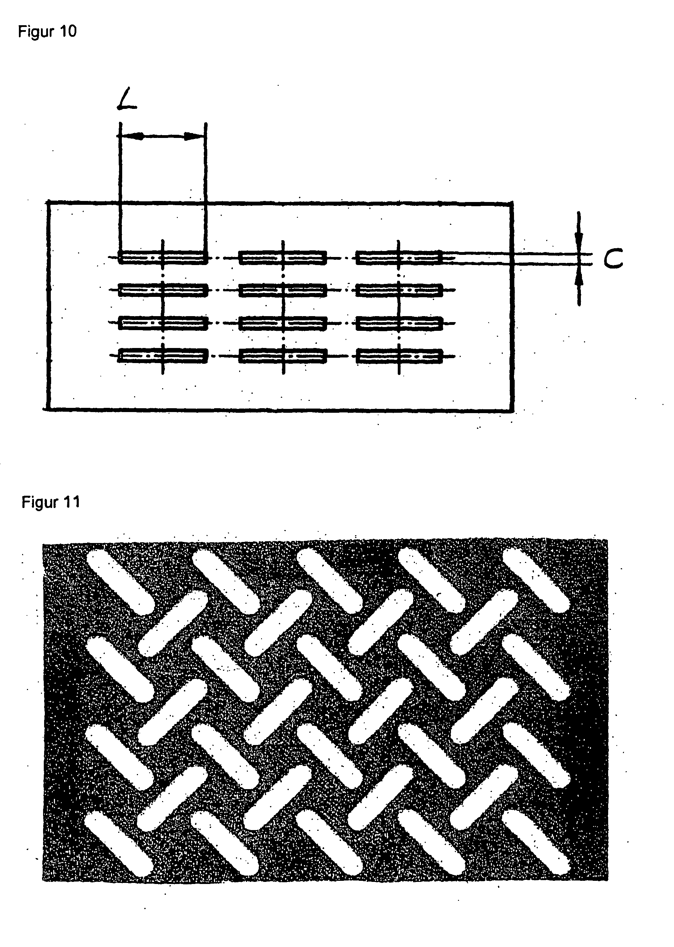

Besonders vorteilhaft an Loch- bzw. Spaltblechen ist, dass die Relativanordnung von erfindungsgemäß geeigneten Sieböffnungen in nahezu beliebiger Art und Weise möglich ist. Weist das Spaltblech nur eine Sorte einer rechteckigen (bzw. einer quadratischen) bzw. einer Langlochform aufweisenden Sieböffnung auf, kommen als Relativanordnung derselben im Spaltblech für das erfindungsgemäße Verfahren insbesondere die gegeneinander versetzte Sieböffnungsanordnung gemäß Figur 7, die ineinander versetzte Sieböffnungsanordnung gemäß Figur 8 (sie ist (unter anderem aus Stabilitätsgründen) erfindungsgemäß ganz besonders bevorzugt), die Sieböffnungsanordnung in geraden Reihen gemäß der Figuren 9 und 10, oder fischgrätähnliche Sieböffnungsanordnungen gemäß Figur 11 in Betracht. Ein weiterer Vorteil von Spaltblechen liegt darin begründet, dass sie bei Produktumstellungen leichter gereinigt werden können und weniger anfällig für ein Verstopfen der Sieböffnungen durch Steckkorn sind. Auch weisen sie in der Regel eine höhere mechanische Stabilität auf.

Im übrigen können erfindungsgemäß geeignete Lochblechsiebe (bzw. Spaltblechsiebe) wie in der DIN 24041 beschrieben gestaltet werden.

Typische Blechdicken d von erfindungsgemäß verwendbaren Lochblechsieben (bzw. Spaltblechsieben) betragen 1 bis 5 mm, vorzugsweise 1 bis 3 mm, besonders bevorzugt 2 bis 3 mm.

Die freie Siebfläche F (die Gesamt(querschnitts)fläche aller in einem Spaltblechsiebboden befindlichen Sieböffnungen) von erfindungsgemäß günstigen Spaltblechsiebböden wird, bezogen auf die Gesamtfläche des Spaltblechsiebbodens in typischer Weise 10 bis 60 %, vorzugsweise 20 bis 50 % und besonders bevorzugt 30 bis 50 % betragen.

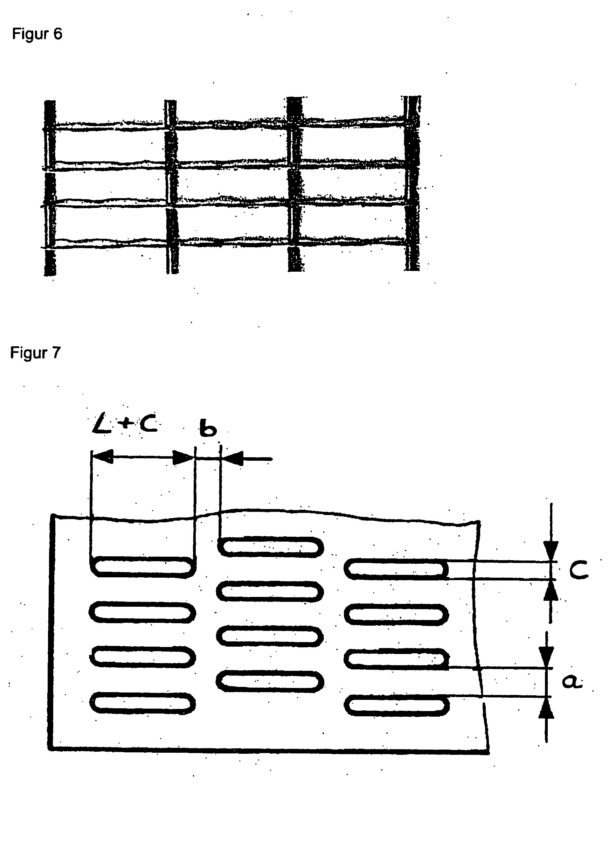

Ein erfindungsgemäß geeignetes Langlochblech (ein erfindungsgemäß geeigneter Langlochsiebboden) mit gegeneinander versetzten Langlöchern gemäß Figur 7 kann z. B. die nachfolgenden Gestaltungsvarianten aufweisen:

| Lochweite C (mm) | Länge L (mm) | Abstand a (mm) | Abstand b (mm) | d (mm) | F (%) |
|---|---|---|---|---|---|
| 1,0 | 19 | 3,0 | 5,0 | 1,5 | 19,8 |
| 1,6 | 18,4 | 2,4 | 5,0 | 1,0 | 31,4 |
| 2,0 | 18 | 10 | 4,5 | 1,25 | 13,3 |
| 2,5 | 17,5 | 3,5 | 5,0 | 1,0 | 32,4 |
| 4,0 | 6,0 | 7,0 | 4,0 | 2,0 | 23,7 |
| 5,0 | 20,0 | 4,0 | 5,0 | 2,0 | 45 |
| 5,0 | 15,0 | 3,0 | 5,0 | 2,0 | 47 |
| 5,0 | 15,0 | 4,0 | 5,0 | 2,0 | 42 |
| 8,0 | 17,0 | 8,0 | 11 | 2,0 | 32 |
| 10,0 | 22,0 | 6,0 | 8,0 | 2,0 | 47 |

Als Werkstoff kommt insbesondere Stahl (z. B. die DIN-Werkstoffe 1.4541 oder 1.4571 sowie Stahl S185 (DIN-Werkstoff 1.0035) gemäß DIN EN 10025 bzw. DIN EN 10088-1) in Betracht.

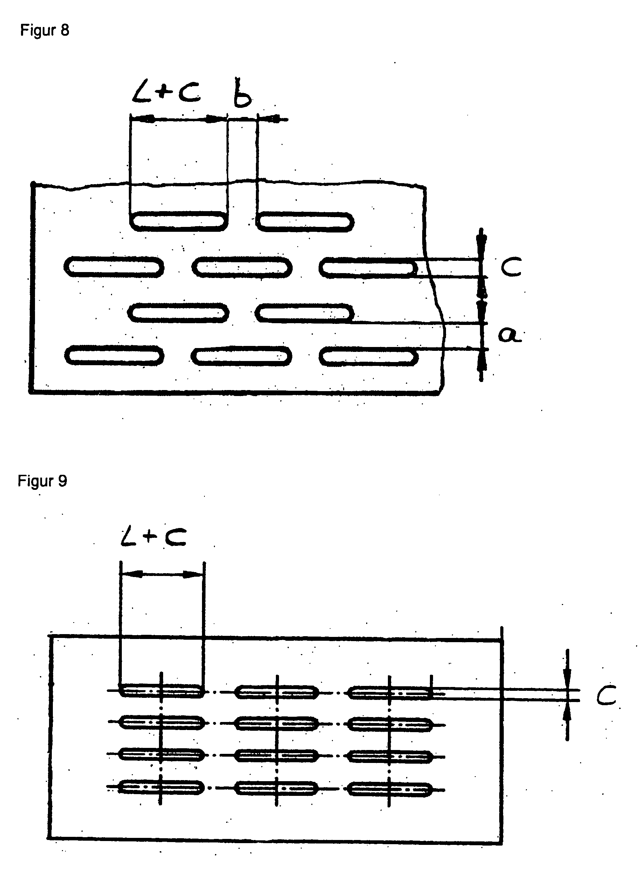

Ein erfindungsgemäß geeignetes Langlochsieb mit ineinander versetzen Langlöchern gemäß Figur 8 kann z. B. die nachfolgenden Gestaltungsvarianten aufweisen:

| Lochweite C (mm) | Länge L (mm) | Abstand a (mm) | Abstand b (mm) | d (mm) | F (%) |
|---|---|---|---|---|---|
| 1,0 | 9 | 2,0 | 4,0 | 1 | 24 |
| 2,0 | 10,5 | 3,0 | 5,5 | 1 | 27 |
| 2,0 | 18,0 | 3,0 | 5,0 | 1 | 31 |
| 4,0 | 18,0 | 3,0 | 5,0 | 1 | 44 |
| 8,0 | 17,0 | 8,0 | 11 | 1 | 32 |

Als Werkstoff kommt insbesondere Stahl (z. B. die DIN-Werkstoffe 1.4541 oder 1.4571) in Betracht.

Im Fall von ringförmigen Schalenkatalysatoren K der Geometrie A x l x H = 7 mm x 4 mm x 3 mm eigenen sich für eine erfindungsgemäße Absiebung von Schalenkatalysatorringzwillingen z. B. Langlochsiebe der vorbeschriebenen Art (insbesondere mit ineinander versetzter Sieböffnungsanordnung) mit C = 5,50 mm und L = 14, 1 mm.
Die Blechdicke kann z. B. 2,2 mm betragen.
Die Stegbreite a zwischen Langlöchern beträgt dabei anwendungstechnisch zweckmäßig 4,0 mm und der Abstand b zweier auf einer gemeinsamen Längslinie aufeinanderfolgenden Langlöchern liegt dabei vorteilhaft bei 5,0 mm. Die freie Siebfläche F beträgt in diesem Fall 36,5 %.

Bei der Ausführung einer erfindungsgemäßen Absiebung erfolgt der Transport des Siebgutes über einen erfindungsgemäß geeigneten Siebboden erfindungsgemäß vorteilhaft parallel zur Vorzugsrichtung L der erfindungsgemäßen Sieböffnungen. In entsprechender Weise erfolgt auch die Aufgabe des Siebgutes auf das Sieb (auf den Siebboden) mit dieser Aufgaberichtung.

Handelt es sich beim erfindungsgemäß verwendeten Siebboden um ein Lochblech mit gestanzten Sieböffnungen, so wird der Stanzgrat in der Regel entfernt und die Umrisslinie der Sieböffnungen anwendungstechnisch zweckmäßig abgerundet. Über die Siebbodendicke ist der Querschnitt einer Sieböffnung normalerweise im wesentlichen konstant (d. h., die Öffnung weist in der Regel einen konstanten Durchgangsquerschnitt auf). Wird der Stanzgrat nicht entfernt, zeigt er normalerweise in Richtung des Siebdurchgangs.

Grundsätzlich kann der Transport des Siebgutes über das Sieb bei einem erfindungsgemäßen Siebverfahren durch eine kreis-, ellipsen- und/oder linearförmige Schwingbewegung des Siebbodens erfolgen. Zu diesem Zweck können für ein erfindungsgemäßes Siebverfahren prinzipiell alle in z. B. Chem.-Ing.-Tech. 56 (1984) Nr. 12, S. 897 bis 907 sowie in Sieben und Siebmaschinen, Grundlagen und Anwendung, Wiley VCH, Paul Schmidt (2003) empfohlenen Siebmaschinen eingesetzt werden. Auch kommen die in den Schriften DE-A 3520614, EP-A 205089 und DE-A 3431337 sowie die in Aufbereitungstechnik 42 (2001) Nr. 7, Seite 345 bis 348 und in Aufbereitungstechnik 41 (2000) Nr. 7, Seite 325 bis 329, beschriebenen Siebmaschinen der Mehrdeck-Bauart für ein erfindungsgemäßes Siebverfahren in Betracht.

Eine zur Durchführung eines erfindungsgemäßen Verfahrens geeignete Gruppe von Siebmaschinen sind die Plansiebe, bei denen das Siebgut als Siebgutmatte linear oder kreisend auf dem Sieb (dem Siebboden) gleitet. Durch das Eigengewicht und die Reibung gegen das Sieb wird die Siebgutmatte geschert. Von Vorteil ist die sehr geringe, meist negativ wirkende Rückvermischung.

Die Schwingbewegung der Siebfläche erfolgt bei Plansieben in ihrer Siebebene. Die Schwingbewegung kann linear (hin und her) oder kreisförmig verlaufen (im ersten Fall spricht man von einem linearen Planschwingsieb). Im ersteren Fall kann sie in Förderrichtung oder quer dazu erfolgen. Durch asymmetrische Beschleunigung kann bei linearer Schwingbewegung in Förderrichtung auch beim horizontalen Sieb der Längstransport des Siebgutes bewirkt werden.

Die Kreisschwingung bietet den Vorteil, stetig eine optimale Beschleunigung aufrechtzuerhalten. Selbstverständlich ist beim erfindungsgemäßen Verfahren auch eine Kombination aus Linear- und Kreisschwinger anwendbar.

Bei Kreisschwingern wird die horizontale Kreisbewegung häufig durch einen Getriebemotor erzeugt. Bei Linearschwingern wird der ganze Siebrahmen (in welchem sich der Siebboden normalerweise ganz generell eingebracht befindet) durch gegenläufige Unwuchtmassen in eine Linearschwingung versetzt. Linearschwinger können sowohl mit horizontalem, als auch mit geneigtem Siebboden angewendet werden. Beim geneigten Siebboden wird das Siebgut durch entsprechende Neigung der Schwingungsebene gegen den Siebboden, entsprechend einer Wurfparabel, aufwärts und zugleich vorwärts geworfen. Die Neigungswinkel können z. B. von -3° bis 25° betragen. 3° bis 4° sind erfindungsgemäß bevorzugt. Erfindungsgemäß geeignet sind zum Beispiel Linearschwingsiebe der Firma RHEWURM GmbH in DE-Remscheid. Rechtecksiebmaschinen sind für einen erfindungsgemäßen Plansiebbetrieb gegenüber Rundsieben in der Regel bevorzugt. Bei ihnen sind normalerweise rechteckige Siebböden in einen gleichfalls rechteckigen Siebrahmen eingebracht.

Vorteilhaft wird für eine erfindungsgemäße Siebabtrennung eine Übereinanderanordnung von Siebböden angewendet, wie sie z. B. im Fall der bereits erwähnten Siebmaschinen der Mehrdeck-Bauart üblich ist.

In diesem Fall wird man erfindungsgemäß zweckmäßig die Schalenkataylsatorringzwillinge (sowie gegebenenfalls sonstige Schalenkatalysatorringmehrlinge) in erfindungsgemäßer Weise mit dem obersten Sieb als Siebrückstand wenigstens teilweise abtrennen. Die erwünschten Schalenkatalysatorringe K sowie gegebenenfalls im Vergleich zu den Schalenkatalysatorringen K feinteiligere Bestandteile des Siebgutes werden hingegen vom obersten Siebboden zum darunter liegenden Siebboden durchgereicht. Dessen Sieböffnungen können z. B. der Lehre der US-A 7,147,011 und der EP-A 1726358 folgend so gestaltet werden, dass die Schalenkatalysatorringe K den Siebrückstand (das Überkorn) und die feinteiligen Bestandteile des Siebgutes den Siebdurchgang (das Unterkorn) bilden. Alternativ zur Lehre der US-A 7147011 und der EP-A 1726358 können bezüglich eines ringförmigen Schalenkatalysators K mit der Geometrie A x I x H unter Maßgabe A ≥ H und der Zielsetzung eines Anfalls der ringförmigen Schalenkatalysatoren K als Überkorn die Sieböffnungen des Siebbodens aber auch so gestaltet werden, dass ihre geschlossene Umrisslinie jeweils wenigstens zwei geradlinige Abschnitte aufweist, die sich im Abstand C* über wenigstens eine Länge L* wie zwei parallele Seiten eines Rechtecks mit den Seitenlängen L* und C* mit der Maßgabe gegenüberstehen, dass jede durch einen auf der Umrisslinie einer Sieböffnung liegenden Umrisslinienpunkt P verlaufende Parallele zur gedachten Rechteckseite mit der Seitenlänge C* keinen weiteren auf der Umrisslinie liegenden Punkt aufweist, dessen Abstand zum Umrisslinienpunkt P > C* beträgt und gleichzeitig die Relationen L* > A ≥ H > C* ≥ (A-I)/2 erfüllt sind.

Bei einer Übereinanderanordnung von Siebböden können sowohl Rundsiebe als auch Rechtecksiebe eingesetzt werden. Die Schwingbewegung wird dabei vorzugsweise so gestaltet, dass der Siebrückstand jeweils zum Umfang des Rundsiebes bzw. Rechtecksiebes hingetragen und dort ausgetragen werden.

Im Fall einer erfindungsgemäßen Verwendung von Siebmaschinen der bereits angesprochenen Mehrdeck-Bauart, wird man mit Vorteil auf einen Mogensen-Sizer® zurückgreifen.

Das System eines Mogensen-Sizers (z. B. eines für eine erfindungsgemäße Abtrennung geeigneten vom Typ "SZ 0534" auf einer Maschine 04711, Baujahr 1997) besteht aus wenigstens zwei übereinanderliegenden, normalerweise unterschiedlich geneigten Siebdecks mit nach unten hin kleiner werdenden Sieböffnungen und zunehmendem Neigungswinkel (zur Horizontalen). In der Regel liegen die Neigungswinkel im Bereich von 5 bis 30°. Für die erfindungsgemäßen Bedürfnisse ist üblicherweise die Anwendung eines Mogensen-Sizers mit zwei Siebdecks ausreichend. Das obere der beiden Siebdecks bewerkstelligt die erfindungsgemäße Zwillingsabtrennung und mit dem nach unten nachfolgenden Siebdeck können die ringförmigen Schalenkatalysatoren K von feinteiligeren Bestandteilen des Siebgutes als Siebüberkorn abgetrennt werden. Bedingt durch die unterschiedliche Neigung der Siebböden (Siebbeläge) wirken deren Sieböffnungen wie Sieböffnungen von geringerer Größe. Deshalb können im Vergleich zu Flachsieben die Sieböffnungen bei im wesentlichen gleich guter Trennschärfe vergleichsweise größer gewählt werden, was erhöhte spezifische Siebleistungen ermöglicht. Charakteristisch für einen Mogensen Sizer ist, dass das Siebgut zunächst aufgelockert wird und nachfolgend die einzelnen Siebdecks fast senkrecht im freien Fall durchströmt. Die erzeugten Grobfraktionen werden jeweils in einem Auslauf des Sizers zusammengefasst und über einen dem Auslauf zugeordneten Auslaufstutzen aus dem Sizer herausgeführt. Ausführliche Darstellungen von Mogensen Sizern finden sich z. B. in Aufbereitungstechnik 42 (2001) Nr. 7, Seite 345 bis 348, und in Aufbereitungstechnik 41 (2000) Nr. 7, Seite 325 bis 329 und der in diesen beiden Schriften zitierten Literatur, aber auch in DE-A 3520614, EP-A 205089 und in DE-A 3431337.

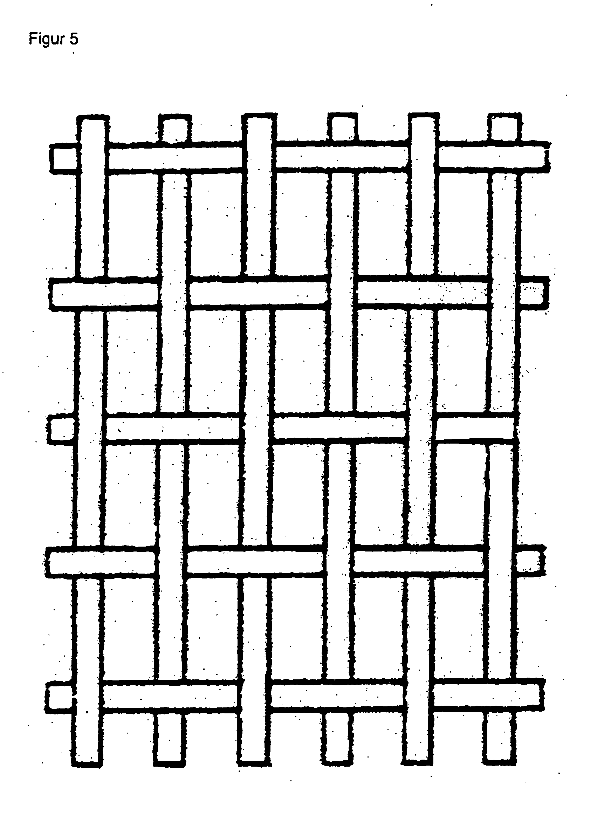

Bei der Herstellung von ringförmigen Schalenkatalysatoren K mit einer ringförmigen Geometrie A x I x H = 8 mm x 5 mm x 6 mm kann z. B. zur erfindungsgemäßen Zwillingsabtrennung ein Zweideck-Mogensen-Sizer vom Typ SZ 0534 (Maschine 04711) mit zwei rechteckigen Siebdecks (z. B. 1340 mm Länge und 490 mm Breite) eingesetzt werden. Der Abstand der beiden Siebböden liegt in der Vertikalen z. B. bei einem Maximalwert von 130 mm. Bei den verwendeten Sieben handelt es sich in erfindungsgemäß zweckmäßiger Weise um Siebgewebe gemäß Figur 5, jedoch mit quadratischen Sieböffnungen im Fall des oberen Siebdecks (die Geometrie der Umrisslinie der Sieböffnungen des oberen Siebdecks beträgt vorteilhaft 10 mm x 10 mm und die Geometrie der Umrisslinie der Sieböffnungen des unteren Siebdecks beträgt vorteilhaft 6 mm x 130 mm; die Dicke des verwobenen Stahldrahtes liegt in typischer Weise bei 1,5 bis 1,7 mm; die Neigung des oberen Siebbodens zur Horizontalen beträgt zweckmäßig 10° und derjenige des unteren Siebbodens zweckmäßig 20°; typische Siebgutdurchsatzströme betragen 300 bis 350 kg/h; im weiteren Verlauf dieser Schrift wird ein solcher Mogensen-Sizer als ein "Mogensen Sizer I" bezeichnet).

Haben die hergestellten ringförmigen Schalenkatalysatoren K die ringförmige Geometrie A x I x H = 7 mm x 4 mm x 7 mm, kann für eine erfindungsgemäße Zwillingsabtrennung ein entsprechend aufgebauter Mogensen-Sizer eingesetzt werden. Die Geometrie der Umrisslinie der Sieböffnungen des oberen Siebdecks beträgt dabei jedoch vorteilhaft 8 mm x 8 mm und die Geometrie der Umrisslinie der Sieböffnungen des unteren Siebdecks beträgt vorteilhaft 5 mm x 130 mm. Dieser Mogensen Sizer soll in dieser Schrift als "Mogensen Sizer II" bezeichnet werden.

Während bei einer erfindungsgemäßen Abtrennung mit Hilfe eines Mogensen Sizers keine zwischen den beiden Siebdecks eingebrachten Siebungshilfsmittel mitverwendet werden, um den oberen und den unteren Siebboden kontinuierlich von Steckkorn freizuhalten, ist es bei einer überwiegend horizontalen Siebfläche in der Regel zweckmäßig, zu diesem Zweck die sogenannte Gummiballklopfung einzusetzen (vgl. Abbildung 12 in Chem.-Ing.Tech. 56 (1984) Nr. 12, Seite 902). Dabei werden Gummibälle auf einen Blindboden gegeben, der sich in einem Abstand Z vom 1,2- bis 1,5-fachen des Gummiballdurchmessers unter dem eigentlichen Sieb (Siebboden) befindet. Die Gummibälle springen auch bei Plansiebmaschinen während des Siebvorgangs (während der Siebung) von unten gegen das Sieb und reinigen dieses örtlich. Ihre Elastizität ist vorteilhaft so bemessen, dass sie im wesentlichen keinen Bruch des Siebgutes bedingen. Der Blindboden ist meist ein Lochblech mit vorzugsweise quadratischen Lochöffnungen. In jedem Fall sind die Lochöffnungen des Blindbodens so beschaffen, dass der Siebdurchgang passieren kann.

Anwendungstechnisch vorteilhaft werden Siebboden (als "Deckebene") und Blindboden (als "Grundebene") mit identischer Gesamtquerschnittsfläche ausgestattet sowie durch vier Seitenwände der Höhe Z zu einem quaderförmigen Seiteneinleger ergänzt, der in einfacher Weise in den Siebrahmen (die Rahmenhöhe überragt den eingelegten Siebeinleger in der Regel um etwa 10 cm) der Siebmaschine eingelegt werden kann. Alternativ zu einer Gummiballklopfung kann eine Siebreinigung während des Siebvorgangs auch durch über und/oder unter dem Siebboden angeordnete Flach- oder Rollenbürsten bewirkt werden.

Die Herstellung von ringförmigen Schalenkatalysatoren K und eine erfindungsgemäße Siebabtrennung können sowohl räumlich voneinander getrennt als auch räumlich unmittelbar ineinander übergehend durchgeführt werden. Letzteres ist z. B. dann zweckmäßig, wenn die Herstellung der ringförmigen Schalenkatalysatoren K wie in der DE-A 2909671, DE-A 102005010645, der EP-A 714700, der DE-A 10325488, der DE-A 10360058, der WO 2004/108267 sowie der Deutschen Anmeldung mit dem Aktenzeichen 102007010422.9 beschrieben erfolgt.

Bei diesen Herstellverfahren wird der ringförmige Trägerformkörper zunächst mit einem flüssigen Bindemittel befeuchtet, anschließend wird durch Inkontaktbringen mit feinteiliger, trockener katalytischer Aktivmasse (z. B. Mulitelementoxidmasse) und/oder feinteiliger trockener Vorläufermasse der katalytischen Aktivmasse an der Oberfläche des befeuchteten ringförmigen Trägerformkörpers eine Schicht (Schale) der vorgenannten feinteiligen Masse angeheftet und anschließend das flüssige Bindemittel aus dem mit der feinteiligen Masse beschichteten ringförmigen Trägerformkörper unter der Einwirkung von Wärme wenigstens teilweise verflüchtigt sowie in der Schale gegebenenfalls enthaltene Vorläufermasse durch thermische Behandlung in die Aktivmasse überführt. In dieser Schrift wird diese Herstellweise als "Coating-Verfahren" bezeichnet.

Als flüssige Bindemittel kommen dabei insbesondere alle diejenigen in Betracht, die in der DE-A 10 2005 010645 und in der EP-A 714700 aufgeführt sind. Dazu zählen insbesondere anorganische und organische Flüssigkeiten sowie Mischungen aus anorganischen und organischen Flüssigkeiten.

Beispielhaft genannt seien Wasser, einwertige Alkohole wie z. B. Methanol und Ethanol, mehrwertige Alkohole wie z. B. Ethylenglycol (bzw. einfach Glycol) und Glycerin, Ether aus vorgenannten ein- und mehrwertigen Alkoholen (z. B. Diethylether, Dibutylether und Glycoldiethylether), Ester aus organischen Carbonsäuren und den vorgenannten ein- und mehrwertigen Alkohlen wie z. B. der Essigsäureethylester, organische Carbonsäuren selbst, wie z. B. Essigsäure, organische Amine, organische Amide, Aminoalkohole (z. B. Ethanolamin), Kohlenwasserstoffe wie z. B. Benzine, Lösungen anorganischer Salze in Wasser und/oder organischen Alkoholen, Lösungen von organischen Fettsäuren in Wasser, Lösungen von Mono- und Oligosacchariden in Wasser, Gemische aus unterschiedlichen Teilmengen der vorgenannten Flüssigkeiten, aber auch Lösungen von Polymerisaten sowie Dispersionen von Polymerisaten.

Bevorzugte flüssige Bindemittel sind Lösungen, die zu 20 bis 90 Gew.-% aus Wasser und zu 10 bis 80 Gew.-% aus einer in Wasser gelösten organischen Verbindung bestehen. Vorzugsweise beträgt der organischen Anteil an der als Bindemittel zu verwendenden wässrigen Lösung 10 bis 50 Gew.-%, besonders bevorzugt 20 bis 30 %. Als organische Komponente des flüssigen Bindemittels eignen sich dabei insbesondere ein- und mehrwertige organische Alkohole wie Glycol, 1,4-Butandiol, 1,6-Hexandiol sowie Glycerin, ein- oder mehrwertige organische Carbonsäuren wie Propionsäure, Oxalsäure, Malonsäure, Glutarsäure sowie Maleinsäure, Aminoalkohole wie Ethanolamin oder Diethanolamin, ein- oder mehrwertige organische Amide wie Formamid, oder Monosaccharide und Oligosaccharide wie Glucose, Fructose, Saccharose oder Lactose. Die Vorteilhaftigkeit derartiger Lösungen als flüssiges Bindemittel liegt unter anderem darin begründet, dass sie in der Regel sowohl die ringförmigen Trägerkörper als auch die auf diese aufzubringende feinteilige Masse zu benetzen vermögen. Als Materialien der ringförmigen Trägerformkörper kommen dabei alle in dieser Schrift sowie alle in der Deutschen Anmeldung mit dem Aktenzeichen 102007010422.9, in der DE-A 10 2005 010 645 und in der EP-A 714 700 genannten Materialien in Betracht. Dazu gehören insbesondere Aluminiumoxid, Siliciumdioxid, Silicate wie Ton, Kaolin, Steatit, Bims, Aluminiumsilicat und Magnesiumsilicat, Siliciumcarbid, Zirkondioxid und Thoriumdioxid.

Mit Vorteil ist die Oberfläche des ringförmigen Trägerformkörpers rau (wie in den vorgenannten drei Schriften empfohlen) da eine erhöhte Oberflächenrauhigkeit in der Regel eine erhöhte Haftfestigkeit der auf der Oberfläche der ringförmigen Trägerformkörpers aufgebrachten Schale an Aktivmasse und/oder Vorläufermasse bedingt.

Desweiteren ist das Trägermaterial bevorzugt unporös (Gesamtvolumen der Poren auf das Volumen des Trägerkörpers bezogen ≤ 1 Vol.-%).

Für eine Durchführung des vorstehend beschriebenen Verfahrens zur Herstellung von ringförmigen Schalenkatalysatoren K eignet sich vor allem das in der DE-A 2909671 offenbarte Verfahrensprinzip (vgl. auch EP-A 714 700 sowie DE-A 10 2005 010 645) unter Verwendung des jeweils erwünschten flüssigen Bindemittels.

D. h., die zu beschichtenden ringförmigen Trägerformkörper werden in einen vorzugsweise geneigten (der Neigungswinkel beträgt in der Regel 30 bis 90 °) rotierenden Drehbehälter (z. B. Drehteller oder Dragierkessel oder Dragiertrommel) gefüllt. Günstige Drehbehälter zu diesem Verwendungszweck sind insbesondere der Hi-Coater vom Typ HCF-100, der Fa. Freund Industrial Co., Ltd,Tokyo (JP) sowie der Hi-Coater vom Typ LH 100, der Fa. Gebrüder Lödige Maschinenbau GmbH, DE-Paderborn.

Der rotierende Drehbehälter führt die ringförmigen Trägerformkörper unter zwei in vorteilhaftem Abstand aufeinanderfolgend angeordneten Dosiervorrichtungen hindurch. Die erste der beiden Dosiervorrichtungen entspricht zweckmäßig einer Düse, durch die die im rotierenden Drehteller (Hi-Coater) rollenden ringförmigen Trägerformkörper mit dem flüssigen Bindemittel kontrolliert befeuchtet werden. Die zweite Dosiervorrichtung befindet sich anwendungstechnisch zweckmäßig außerhalb des Zerstäubungskegels des eingesprühten flüssigen Bindemittels und dient dazu, die feinteilige Aktivmasse (z. B. eine feinteilige Multielementoxidaktivmasse) und/oder die feinteilige Vorläufermasse zuzuführen (z. B. über eine Schüttelrinne). Die kontrolliert befeuchteten ringförmigen Trägerformkörper nehmen die zugeführte feinteilige Masse (das feinteiligen Pulver) auf, das sich durch die rollende Bewegung auf der äußeren Oberfläche der ringförmigen Trägerformkörper zu einer zusammenhängenden Schale verdichtet (im inneren Kreis der ringförmigen Trägerformkörper findet eine solche verdichtende Bewegung nicht statt, weshalb dieser im wesentlichen unbeschichtet bleibt).

Bei Bedarf durchläuft der so grundbeschichtete ringförmige Trägerformkörper im Verlauf der darauffolgenden Umdrehung wiederum die Sprühdüse, wird dabei kontrolliert befeuchtet (gegebenenfalls mit einem anderen flüssigen Bindemittel), um im Verlauf der Weiterbewegung eine weitere Schicht (einer gegebenenfalls anderen) feinteiligen Aktivmasse und/oder Vorläufermasse aufnehmen zu können usw. (eine Zwischentrocknung ist in der Regel nicht erforderlich). Die wenigstens teilweise Entfernung des verwendeten flüssigen Bindemittels kann z. B. gemäß der Lehre der EP-A 714 700 oder der Lehre der DE-A 10 2005 010 645 folgend durch abschließende Wärmezufuhr, z. B. durch Einwirkung von heißen Gasen wie $N_2$ oder Luft erfolgen (diese werden durch räumlich voneinander getrennt angebrachte netzartig gestaltete Wandelemente des Drehtellers, bzw. Dragierkessels, bzw. der Dragiertrommel (allgemein Drehbehälters) zu- und abgeführt).

Von Bedeutung für die beschriebene Ausführungsform des Beschichtungsverfahrens ist, dass die Befeuchtung der zu beschichtenden Oberfläche der ringförmigen Trägerformkörper in kontrollierter Weise vorgenommen wird. Kurz ausgedrückt heißt dies, dass man die Trägeroberfläche zweckmäßig so befeuchtet, dass diese zwar flüssiges Bindemittel adsorbiert aufweist, auf der Trägeroberfläche eine solche visuell aber nicht in Erscheinung tritt. Ist die Trägerformkörperoberfläche zu feucht, agglomeriert die feinteilige Aktiv- und/oder Vorläufermasse zu getrennten Agglomeraten, anstatt auf die Oberfläche aufzuziehen. Detailliertere Angaben hierzu finden sich in der DE-A 2909671, in der EP-A 714 700 sowie in der DE-A 10 2005 010 645. Letzteres gilt insbesondere auch für die abschließende wenigstens teilweise Entfernung des verwendeten flüssigen Bindemittels. Ein weiterer Vorzug der beschriebenen Verfahrensweise besteht nämlich darin, dass diese Entfernung in vergleichsweise kontrollierter Weise z. B. durch Verdampfen und/oder Sublimieren vorgenommen werden kann. Im einfachsten Fall kann dies wie bereits ausgeführt durch die Einwirkung heißer Gase entsprechender Temperatur (häufig 50 bis 150 °C) erfolgen. Eine solche Einwirkung heißer Gase kann sowohl eine vollständige Trocknung oder nur eine Vortrocknung bewirken. Die Endtrocknung kann dann beispielsweise in einer Trockenvorrichtung beliebiger Art (z. B. in einem Bandtrockner) und/oder oder erst im Katalysatorfestbett des Rohrbündelreaktors befindlich erfolgen, wie es z. B. die DE-A 10 2005 010 645 empfiehlt.

Der Übergang zu einer erfindungsgemäßen Zwillingsabtrennung durch Sieben kann nun in einfacher Weise z. B. wie folgt gestaltet sein. Die im Drehbehälter befindliche Produktionscharge an ringförmigem Schalenkatalysator kann durch Öffnen einer über einem Trichter befindlichen Entleerungsklappe über den Trichter entleeren. Der Auslauf des Trichters wird nun unmittelbar in ein gegen die Horizontale leicht geneigtes (Austrags-) Rohr (der Neigungswinkel kann z. B. von -3° bis 25° betragen; 3° bis 5° sind bevorzugt) fortgeführt.

Das Austragsrohr weist in typischer Weise eine Länge von 1200 mm und einen Innendruchmesser von z. B. 100 mm auf. Im übrigen ist es als Linearschwingsieb ausgestaltet. Zu diesem Zweck enthält es einen sich über die gesamte Rohrlänge erstreckenden Siebboden eingebaut, der das Rohrinnere in ein oberes (oberhalb des Siebbodens befindliches) und in ein unteres (unterhalb des Siebbodens befindliches) Halbrohr aufteilt. Durch Taumelbewegungen des Drehbehälters entleert sich die im Drehbehälter befindliche Produktionscharge in das obere Halbrohr des den Siebboden enthaltenden Austragsrohres.

Im Austragsrohr wird es zu dessen Ende transportiert (typisch: 80 kg Schalenkatalysatorringe pro 30 Minuten). Auf dem Weg der Produktionscharge durch das Austragsrohr findet über den Siebboden des Austragsrohres die erfindungsgemäße Zwillingsabtrennung statt. Die erwünschten ringförmigen Schalenkatalysatoren K bilden den Siebdurchgang, der am Ende des unteren Halbrohres ausgetragen wird, während der Austrag der abgetrennten Zwillinge am Ende des oberen Halbrohres aus selbigem erfolgt.

Handelt es sich bei den ringförmigen Schalenkatalysatoren K um solche der Geometrie A x I x H = 7 mm x 4 mm x 3 mm, so wird man als Siebboden im vorgenannten Austragsrohr in vorteilhafter Weise ein Langlochsieb mit ineinander versetzten Langlöchern gemäß Figur 8 einsetzen. Erfindungsgemäß vorteilhaft wird C = 5,50 mm und L = 14,1 mm betragen. Eine Blechdicke von 2,2 mm ist dabei ebenso zweckmäßig wie eine Stegbreite a von 4,0 mm und ein Abstand b von 5,0 mm. Die freie Siebfläche F beträgt in diesem Fall 36,5 %.

Ein Hi-Coater vom Typ LH 100 der Fa. Gebrüder Lödige Maschinenbau GmbH, DE-Paderborn, der ein wie eben beschriebenes Austragsrohr zur Zwillingsabtrennung aufweist, soll in dieser Schrift als "Abtrenn-Hi-Coater I" bezeichnet werden.

Die erfindungsgemäß abgetrennten Schalenkatalysatorringzwillinge werden in der Regel nicht entsorgt, sondern aufgearbeitet. D. h., üblicherweise wird man versuchen, die in der Aktivmasse derselben enthaltenen Elemente wiederzugewinnen.

Eine erfindungsgemäße Siebabtrennung wird in der Regel an Luft durchgeführt (insbesondere im Fall aller in dieser Schrift beispielhaft aufgeführten Multielementoxidkatalysatoren).

Bei stark hygroskopischen oder sauerstoffempfindlichen Schalenkatalysatoren K bzw. Aktivmassen derselben, kann die Zwillingsabsiebung auch unter Ausschluß von Feuchtigkeit und/oder Sauerstoff (z. B. unter $N_2$) erfolgen. Von der Siebabtrennung werden die ringförmigen Schalenkatalysatorformkörper K in der Regel unmittelbar einem luftdicht verschließbaren Behältnis zugeführt, in welchem sie gelagert werden können. Aus diesem Behältnis (z. B. ein mit einer Polypropyleneinhüllenden ausgeschlagenes Fass) können sie dann z. B. zum Zweck einer strukturierten Befüllung von Reaktionsrohren der Lehre der DE-A 10 2004 023 249 folgend entnommen und in das ins Auge gefasste Reaktionsrohr eingebracht werden.

Alternativ zum Coating-Verfahren zur Herstellung von Schalenkatalysatoren K wird man zur Herstellung von Schalenkatalysatoren K die ringförmigen Trägerformkörper häufig auch mit einer Suspension von feinteiliger Aktivmasse und/oder feinteiliger Vorläufermasse besprühen.

Erfindungsgemäß vorteilhaft wird man dabei so vorgehen, wie es in der DE-A 4006935 und DE-A 10344844 beschrieben ist. Die z. B. wässrige Suspension der feinteiligen Aktivmasse und/oder feinteiligen Vorläufermasse, die, um die Qualität der Beschichtung der ringförmigen Trägerformkörper zu verbessern, in der Regel organische Bindemittel (in der Regel Copolymerisate, z. B. solche auf der Basis von Vinylacetat/Vinyllaurat, oder von Vinylacetat/Acrylat, oder von Styrol/Acrylat, oder von Vinylacetat/Ethylen) zugesetzt enthält (z. B. in Form einer wässrigen Polymerisatdispersion), wird bei erhöhter Temperatur so lange auf die ringförmigen Trägerformkörper aufgesprüht, bis der gewünschte Aktivmassenanteil am Katalysatorgesamtgewicht erreicht ist. Hierzu eignen sich insbesondere Wirbelbett- bzw. Fließbettapparate. In diesen werden die ringförmigen Trägerformkörper in einem aufsteigenden Gasstrom (z. B. heiße Luft) fluidisiert. Die Apparate bestehen meist aus einem konischen oder kugelförmigen Behälter, bei dem das fluidisierende Gas von unten oder von oben über ein Zentralrohr eingeführt wird. Die Suspension wird über Düsen von oben, seitlich oder von unten in die Wirbelschicht der ringförmigen Trägerformkörper eingesprüht. Vorteilhaft ist der Einsatz eines mittig bzw. konzentrisch um das Zentralrohr angeordneten Leitrohrs. Innerhalb des Leitrohrs herrscht eine höhere Gasgeschwindigkeit, welche die ringförmigen Trägerformkörper nach oben transportiert. Im äußeren Ring liegt die Gasgeschwindigkeit nur wenig oberhalb der Lockerungsgeschwindigkeit. So werden die ringförmigen Katalysatorformträger kreisförmig vertikal bewegt.

Nähere Einzelheiten über dieses "Aufsprüh-Verfahren" zur Herstellung von ringförmigen Schalenkatalysatoren K finden sich z. B. in der DE-A 10344844 sowie in der DE-A 4006935. Letztere offenbart auch einen diesbezüglich besonders geeigneten Fließbettapparat.

Im übrigen ist eine Produktionscharge von Schalenkatalysatoren K im Sinne dieser Erfindung eine solche Produktionsmenge von Schalenkatalysatoren K, die den Bedarf von wenigstens zwei (besser wenigstens 10, häufig wenigsten 50, meist wenigstens 100, vielfach wenigsten 200 oder wenigstens 500) Reaktionsrohren im Rohrbündelreaktor zu decken vermag.

Ringförmige Schalenkatalysatoren K umfassen unter anderem solche Schalenkatalysatoren, deren Aktivmasse ein Multielementoxid der allgemeinen Formel I,

$$Mo_{12}Bi_aFe_bX_c^1X_d^2X_e^3O_y \qquad (I),$$

mit

$X^1$ = Co und/oder Ni,
$X^2$ = Si und/oder Al,
$X^3$ = Li, Na, K, Cs und/oder Rb,
$0,2 \leq a \leq 1$,
$2 \leq b \leq 10$,
$0,5 \leq c \leq 10$,
$0 \leq d \leq 10$,
$0 \leq e \leq 0,5$, und
y = eine Zahl, die (unter der Voraussetzung der Ladungsneutralität) durch die Wertigkeit und Häufigkeit der von Sauerstoff verschiedenen Elemente in I bestimmt wird,

ist.

Die Herstellung solcher ringförmigen Schalenkatalysatoren K kann mit Vorteil gemäß der Lehre der DE-A 100 49 873 erfolgen. Dazu erzeugt man aus Ausgangsverbindungen der elementaren Konstituenten der katalytisch aktiven Oxidmasse ein inniges Trockengemisch, das bei 150 bis 350 °C unter Erhalt einer Vorläufermasse thermisch behandelt wird. Beim Coating-Verfahren wird unter Verwendung von Wasser als Bindemittel eine Schicht der Vorläufermasse auf den ringförmigen Trägerformkörpern angeheftet und der anfasstrockene beschichtete ringförmige Trägerformkörper anschließend bei 400 bis 600 °C calciniert.

Diese Schalenkatalysatoren eignen sich insbesondere für die gasphasenkatalytische Partialoxidation von Propylen zu Acrolein und von iso-Buten zu Methacrolein. Vorteilhafte Partialoxidationsbedingungen finden sich ebenfalls in der DE-A 10049873.

Das erfindungsgemäße Verfahren ist ferner relevant bei der Herstellung von ringförmigen Schalenkatalysatoren K, die als aktive Schale ein Multielementoxid aufweisen, das die Element V, Sb sowie wenigstens ein Element aus Mo und W enthält.

Die Herstellung solcher Schalenkatalysatoren K offenbart z. B. die WO 2007/00922. Bevorzugtes Herstellverfahren ist auch hier das Vorläufermasse-Coating-Verfahren. Es kann aber ebenso das Aktivmasse-Coating-Verfahren angewendet werden. Derartige Schalenkatalysatoren K eignen sich insbesondere für heterogen katalysierte partielle Ammoxidationen organischer Ausgangsverbindungen. Beispiele hierfür sind die Herstellung von Methylbenzonitrilen und Benzodinitrilen aus Xylol. Günstige Partialammoxidationsbedingungen offenbart diesbezüglich ebenfalls die WO 2007/009922.

Von besonderer Bedeutung ist das erfindungsgemäße Verfahren auch dann, wenn es sich um solche ringförmigen Schalenkatalysatoren K handelt, deren katalytisch aktive Schale eine Multielementoxidaktivmasse der allgemei-nen Formel II,

$$Mo_{12}V_aX_b^1X_c^2X_d^3X_e^4X_f^5X_g^6O_n \qquad (II),$$

mit

$X^1$ =    W, Nb, Ta, Cr und/oder Ce,
$X^2$ =    Cu, Ni, Co, Fe, Mn und/oder Zn,
$X^3$ =    Sb und/oder Bi,
$X^4$ =    eines oder mehrere Alkalimetalle (Li, Na, K Rb, Cs) und/oder H,
$X^5$ =    eines oder mehrere Erdalkalimetalle (Mg, Ca, Sr, Ba),
$X^6$ =    Si, Al, Ti und/oder Zr,

a = 1 bis 6,

b = 0,2 bis 4,

c = 0 bis 18, vorzugsweise 0,5 bis 18,

d = 0 bis 40,

e = 0 bis 2,

f = 0 bis 4,

g = 0 bis 40, und

n = eine Zahl, die durch die Wertigkeit und Häufigkeit der von Sauerstoff verschiede nen Elemente in II bestimmt wird,

ist.

Sie eignen sich vor allem für eine heterogen katalysierte partielle Gasphasenoxidation von Acrolein zu Acrylsäure. Mit Vorteil sind hier die ringförmigen Schalenkatalysatoren nach dem Verfahren der DE-A 10 2005 010645, der DE-A 10325488, der DE-A 10360058, der DE-A 10350822, der DE-A 10 2004 025 445, der DE-A 10 2007 010 422, der US 2006/0205978 sowie der EP-A 714 700 und den Verfahren des in diesen Schriften zitierten Standes der Technik erhältlich. Besonders bevorzugtes Herstellverfahren ist das Aktivmassen-Coating-Verfahren gemäß der EP-A 714 700. Gemäß der Lehre der Deutschen Anmeldung mit dem Aktenzeichen102007010422.9 kann dabei der aufgebrachten Aktivmasse zusätzlich ein feinteiliges Molybdänoxid beigemischt werden, um die Standzeit zu verlängern. Die vorgenannten Schriften offenbaren zusätzlich besonders günstige Bedingungen für eine heterogen katalysierte partielle Gasphasenoxidation von Acrolein zu Acrylsäure.

Die Aktivmassenschalendicke wird hier vor allem 10 bis 1000 $\mu$m, bevorzugt 50 bis 500 $\mu$m und besonders bevorzugt 150 bis 250 $\mu$m betragen. Besonders günstig sind die beispielhaften Ausführungsformen der EP-A 714 700. Bevorzugte Ringgeometrie ist diejenige mit A x I x H = 7 mm x 4 mm x 3 mm.

Weiterhin ist das erfindungsgemäße Verfahren von besonderer Relevanz im Fall von ringförmigen Schalenkatalysatoren K, deren Aktivmasse ein Multielementoxid ist, das als von Sauerstoff verschiedene Elemente neben den Elementen Mo und V wenigstens eines der beiden Elemente Te und Sb und wenigstens eines der Elemente aus der Gruppe umfassend Nb, Pb, Ta, W, Ti, Al, Zr, Cr, Mn, Ga, Fe Ru, Co, Rh, Ni, Pd, Pt, La, Bi, B, Ce, Sn, Zn, Si, Na, Li, K, Mg, Ag, Au und In in Kombination enthalten.

Ihre Herstellung offenbart z. B. die WO 2004/108267. Sie eignen sich u. a. als Katalysatoren für die heterogen katalysierte partielle Gasphasenoxidation von Acrolein zu Acrylsäure, von Propan zu Acrolein und/oder Acrylsäure, von iso-Butan zu Methacrolein und/oder Methacrylsäure sowie für die Ammoxidation von Propan zu Acrylnitril und von iso-Butan zu Methacrylnitril (vgl. auch DE-A 10 2007 025 869).

Das erfindungsgemäße Verfahren sollte aber auch im Fall von ringförmigen Schalenkatalysatoren K angewendet werden, deren Aktivmasse elementares Silber auf oxidischen ringförmigen Trägerkörpern aufweist. Solche ringförmigen Schalenkatalysatoren K eignen sich insbesondere für eine heterogen katalysierte partielle Gasphasenoxidation von Ethylen zu Ethylenoxid (vgl. z. B. die EP-A 496 470). Als Trägerformkörper kommen vor allem solche in Betracht, die zu wenigstens 80 Gew.-% aus Aluminiumoxid (z. B. $Al_2O_3$) bestehen. Als elementares Silber in ihrer auf einem oxidischen ringförmigen Trägerformkörper aufgebrachten Aktivmasse enthaltende Trägerkatalysatoren für eine heterogen katalysierte partielle Gasphasenoxidation von Ethylen zu Ethylenoxid seien auch die ringförmigen Trägerkatalysatoren der EP-A 619 142, EP-A 624 398, EP-A 804 289 und EP-A 937 498 hervorgehoben. Für alle diese ringförmigen Schalenkatalysatoren K kommt das erfindungsgemäße Verfahren als geeignet in Betracht.

Günstige Ringgeometrien A x I x H sind dabei unter anderem die Ringgeometrien 8,5 mm x 3,2 mm x 8,5 mm, sowie 8,5 mm x 3,4 mm x 8,5 mm und 8 mm x 3 mm x 8 mm bzw. 7,7 mm x 3 mm x 5 mm.

Weiterhin hat das erfindungsgemäße Verfahren erhöhte Relevanz im Fall von ringförmigen Schalenkatalysatoren K, deren Aktivmasse wenigstens ein Multielementoxid ist, das zu wenigstens 60 Gew.-% aber zu nicht mehr als 99 Gew.-% oxidische Einheiten $TiO_2$ und zu wenigstens 1 Gew.-% aber zu nicht mehr als 40 Gew.-% oxidische Einheiten $V_2O_5$ enthält. Häufig enthalten die vorgenannten Multielementoxidaktivmassen noch bis zu 1 Gew.-% Cs, bis zu 1 Gew.-% P und bis zu 10 Gew.-% oxidische Einheiten $Sb_2O_3$ Zusätzlich können sie weitere die Aktivität und Selektivität des Schalenkatalysators K promovierende Promotoren enthalten.

Diese ringförmigen Schalenkatalysatoren K eignen sich insbesondere für die heterogen katalysierte partielle Gasphasenoxidation von aromatischen Kohlenwasserstoffe wie Benzol, Naphthalin, Xylolen, Toluol und/oder Durol. Man kann auf diese Weise z. B. Benzoesäure, Maleinsäureanhydrid, Phthalsäureanhydrid, Isophthalsäure, Terephthalsäure und/ oder Pyromellithsäureanhydrid erhalten. Bevorzugtes Herstellverfahren solcher ringförmigen Schalenkatalysatoren K ist das Suspensions-Aufsprüh-Verfahren. Die Herstellung solcher Schalenkatalysatoren K sowie besonders günstige Partialoxidationsbedingungen offenbaren z. B. die DE-A 19839001, die DE-A 10344844, die DE-A 4006935, die DE-A 19823262 und die DE-A 19823275.

Günstige für solche Schalenkatalysatoren geeignete V und Ti enthaltende Multielementoxidaktivmassen offenbaren auch die Schriften US-A 6528683, US-A 6586361 und US-A 6362345, einschließlich günstiger Bedingungen für eine heterogen katalysierte partielle Gasphasenoxidation von o-Xylol und/oder Naphthalin zu Phthalsäureanhydrid (wie stets in dieser Schrift umfasst der Begriff Partialoxidationsbedingungen auch die Strukturierung (Beschickung) des Katalysatorfestbetts in den Reaktionsrohren). Für vorgenannte Partialoxidationen geeignete Ringgeometrien der Schalenkatalysatoren K sind z. B. die Ringgeometrien (A x I x H) 7 mm x 4 mm x 7 mm, 8 mm x 5 mm x 6 mm, 7 mm x 4 mm x 4 mm, 8 mm x 4 mm x 6 mm, sowie 8 mm x 3 mm x 6 mm.

Das erfindungsgemäße Verfahren ist aber auch hervorragend geeignet für die in der DE-A 10 2005 055 827 offenbarten ringförmigen Mo und Fe enthaltenden Multielementoxidschalenkatalysatoren, die insbesondere zur Oxidation von Methanol zu Formaldehyd geeignet sind.

Im übrigen wird die Katalysatorfestbettbeschickung der Reaktionsrohre des Rohrbündelreaktors wie in der Deutschen Anmeldung mit dem Aktenzeichen 102007017080.9 beschrieben erfolgen.

Beispiele

A) Heterogen katalysierte partielle Gasphasenoxidation von Acrolein zu Acrylsäure in einem Thermorohr

I. Beschickung eines Thermorohres mit einem ringförmige Schalenkatalysatoren umfassenden Katalysatorfestbett, wobei vorab der Einbringung der ringförmigen Schalenkatalysatoren eine Zwillingsabtrennung durchgeführt worden ist

Das Thermorohr (V2A Stahl; 33,7 mm Außendurchmesser, 2 mm Wandstärke, 29,7 mm Innendurchmesser, Länge: 350 cm, sowie eine in der Thermorohrmitte zentrierte Thermohülse (10 mm Außendurchmesser) zur Aufnahme eines stationären Multithermoelements mit dem die Temperatur im Thermorohr auf seiner gesamten Länge ermittelt werden kann) wurde von oben nach unten wie folgt beschickt:

Abschnitt 1: 20 cm Länge Steatitringe ST der Geometrie 7 mm x 4 mm x 3 mm (A x I x H) als Vorschüttung (Steatit C 220 der Fa. CeramTec);

Abschnitt 2: 90 cm Länge Katalysatorfestbettbeschickung mit einem homogenen Gemisch aus 30 Gew.-% an Steatitringen ST, 30 Gew.-% ringförmigen Schalenkata- lysatoren K 1 gemäß Ausführungsbeispie 2 der WO 2004/108267, je- doch mit vorheriger Zwillingsabtrennung (7,1 mm x 4,0 mm x 3,2 mm; $Mo_{12}$ $V_3W_{1,2}Cu_{2,4}O_x$; die Beschichtung der ringförmigen Trägerform- körper und die Zwilling-abtrennung erfolgten mit einem Hi-Coater I) und 40 Gew.-% an kugelförmigen Schalenkatalysatoren KU1 (herge- stellt wie die ringförmigen Schalenkatalysatoren K1 jedoch mit kugel- förmigen Trägerformkörpern aus Steatit mit einem Durchmesser von 4 - 5 mm sowie unter Verwendung von Wasser als Bindemittel und ei- nem Gewichtsanteil von 20 Gew.-% der Aktivmassenschale;

Abschnitt 3: 50 cm Länge Katalysatorfestbettbeschickung mit einem homogenen Gemisch aus 20 Gew.-% an Steatitringen ST, 40 Gew.-% ringförmigen Schalenkata- lysatoren K1 (mit vorheriger Zwillingsabtrennung) und 40 Gew.-% ku- gelförmigen Schalenkatalysatoren KU1;

Abschnitt 4: 70 cm Länge Kataylsatorfestbettbeschickung mit einem homogenen Gemisch aus 60 Gew.-% ringförmigen Schalenkatalysatoren K1 (mit vorheriger Zwil- lingsabtrennung) und 40 Gew.-% kugelförmigen Schalenkatalysatoren KU1; und

Abschnitt 5: 120 cm Länge Katalystorfestbettbeschickung mit nur kugelförmigem Schalenkataly- sator KU1.

Von oben nach unten wurden die ersten 175 cm mittels eines im Gegenstrom gepumpten Salzbades A thermostatisiert, das mit der Temperatur $T_w^A$ zugeführt wurde.

Die zweiten 175 cm wurden mittels eines im Gegenstrom gepumpten Salzbades B thermostatisiert, das mit der Temperatur $Tw^B$ zugeführt wurde. Beide Salzbäder waren ein Gemisch aus 53 Gew.-% Kaliumnitrat, 40 Gew.-% Natriumnitrit und 7 Gew.-% Natriumnitrat.

Das vorstehend beschriebene Thermorohr wurde mit einem Reaktionsgaseingangsgemisch der nachfolgenden Gehalte kontinuierlich beschickt:

|         |                     |
|---------|---------------------|
| 4,8 Vol.-% | Acrolein,          |
| 0,5 Vol.-% | Acrylsäure,        |
| 0,2 Vol.-% | Propylen,          |
| 5,5 Vol.-% | molekularer Sauerstoff, |
| 0,5 Vol.-% | CO                 |

(fortgesetzt)

| | |
|---|---|
| 1,1 Vol.-% | $CO_2$, |
| 5,7 Vol.-% | Wasser, und |
| 81,5 Vol.-% | Stickstoff. |

Das Reaktionsgasgemisch durchströmte das Thermorohr von oben nach unten. Der Druck am Eingang des Thermorohres betrug 2,0 bar (absolut). Die Belastung des Katalysatorfestbetts mit Acrolein betrug 140 Nl/l•h. $T_w^A$ wurde auf 275 °C und $T_w^B$ auf 279 °C eingestellt. Das Reaktionsgaseingangsgemisch war auf 220 °C vorerwärmt.

Bei einem auf den einmaligen Durchgang des Reaktionsgasgemischs durch das Thermorohr bezogenen Umsatz des Acroleins von ca. 99,5 mol-% und einer Selektivität der Acrylsäurebildung von 95,0 mol-% betrug die Heißpunkttemperatur im Thermorohr 323 °C. Die Position des Heißpunktmaximums war in Strömungsrichtung des Reaktionsgases 45 cm nach Beginn des Abschnitts 2.

II. Wiederholung der Partialoxidation aus A) I, jedoch unter gezielter Dotierung des in das Thermorohr eingebrachten Katalysatorfestbetts mit einem bei der Herstellung der ringförmigen Schalenkatalysatoren K1 im Rahmen der Zwillingsabtrennung angefallenen Tandem-Zwilling (die Gesamtmenge der abgetrennten Zwillinge belief sich auf 0,24 Gew.-%)

Das Katalysatorfestbett wurde wie in A) I beschickt. In Strömungsrichtung des Reaktionsgases 45 cm nach Beginn des Abschnitts 2 wurde zwischen die Innenwand des Thermorohres und die Außenwand der Thermohülse jedoch gezielt ein Tandem-Zwilling (0,02 Gew.-% bezogen auf das insgesamt eingebrachte Gewicht an ringförmigen Schalenkatalysatoren K1) so platziert, dass seine Längsachse in die Strömungsrichtung des Reaktionsgases wies. Im übrigen wurde wie in A) I verfahren und die Thermorohrbefüllung auch demgemäß zu Ende geführt.

Bei unveränderter Position der Heißpunkttemperatur erhöhte sich diese auf 325 °C.

III. Wiederholung der Partialoxidation aus A) I, jedoch unter gezielter Dotierung des in das Thermorohr eingebrachten Katalysatorfestbetts mit einem bei der Herstellung der ringförmigen Schalenkatalysatoren K1 im Rahmen der Zwillingsabtrennung angefallenen annellierten Zwilling

Das Katalysatorfestbett wurde wie in A) I beschickt. Als in Strömungsrichtung des Reaktionsgases blickend die ersten 70 cm der Reaktionsrohrlänge noch unbeschickt und die übrige Reaktionsrohrlänge bereits beschickt war, wurde in Strömungsrichtung 40 cm nach Beginn des Abschnitts 2 zwischen die Innenwand des Thermorohres und die Außenwand der Thermohülse ein annellierter Zwilling eingeklemmt (eingekeilt). Im übrigen wurde wie in A) I verfahren und die Thermorohrbefüllung nach der durchgeführten Einkeilung auch demgemäß zu Ende geführt.

Die Position des Heißpunktmaximums lag jetzt bereits (in Strömungsrichtung des Reaktionsgases) 40 cm nach Beginn des Abschnitts 2. Sie betrug jetzt 324 °C.

B) Heterogen katalysierte partielle Gasphasenoxidation von Acrolein zu Acrylsäure in einem Arbeitsrohr (das durch das Thermorohr in A) I abgebildet werden soll)

Ein Arbeitsrohr (V2A Stahl; 30 mm Außendurchmesser; 2 mm Wandstärke, 26 mm Innendurchmesser, 350 cm Länge) wurde wie nachfolgend beschrieben mit einem ringförmigen Schalenkatalysatoren K1 (mit vorheriger Zwillingsabtrennung) umfassenden Katalysatorfestbett beschickt. Zur Ermittlung des Verlaufs der Reaktionstemperatur im Arbeitsrohr enthielt dieses zentriert unmittelbar ein Multithermoelement eingeführt (Außendurchmesser = 4 mm; im großtechnischen Rohrbündelreaktor ist diese Vorgehensweise nicht möglich; ein großtechnisches Arbeitsrohr (es enthält kein Thermoelement) würde daher eine insgesamt etwas größere Gesamtaktivmassenmenge enthalten und deshalb für den gleichen Acroleinumsatz um 1 °C tiefer liegende $T_w^A$, $T_w^B$ erfordern).

I Ideale (ohne Zwilling) Beschickung des Arbeitsrohres mit dem Katalysatorfestbett (von oben nach unten)

Abschnitt 1: 20 cm Länge Steatitringe ST;

Abschnitt 2: 90 cm Länge Homogenes Gemisch aus 70 Gew.-% ringförmigen Schalenkatalysato- ren K1 und 30 Gew.-% Steatitringen ST;

Abschnitt 3: 50 cm Länge Homogenes Gemisch aus 80 Gew.-% ringförmigen Schalenkatalysato- ren K1 und 20 Gew.-% Steatitringen ST;

Abschnitt 4: 190 cm Länge nur ringförmiger Schalenkatalysator K1.

In diesem Arbeitsrohr wurde unter den gleichen Bedingungen wie im Thermorohr unter A) I eine heterogen katalysierte partielle Gasphasenoxidation von Acrolein zu Acrylsäure betrieben (aus den bereits angesprochenen Gründen betrug $T_W^A$ = 276 °C und $T_W^B$ = 280 °C, um demselben Acroleinumsatz von 99,5 mol-% zu erzielen).

Die Heißpunkttemperatur lag wie im Thermorohr bei 323 °C. Die Position des Heißpunktmaximums war wie in A) I in Strömungsrichtung des Reaktionsgases 45 cm nach Beginn des Abschnitts 2. II. In gleicher Weise wie in A) II das Thermorohr, wurde die Katalysatorfestbettbeschickung des Arbeitsrohres mit einem Tandem-Zwilling dotiert.

Im Ergebnis stellte sich bei gleich bleibender Lage des Heißpunktmaximums eine um 2 °C erhöhte Heißpunkttemperatur von 325 °C ein.

III. Die Katalysatorfestbettbeschickung wurde wie in B) I vorgenommen, in die den Abschnitt 4 bildende Portion von ringförmigen Schalenkatalysatoren 1 wurde jedoch vorab ihrer Entleerung in das Arbeitsrohr in zufälliger Weise ein Tandem-Zwilling zugegeben (nach der Zugabe wurde die Portion durchgeschüttelt).

Die resultierenden Partialoxidationsergebnisse waren unter ansonsten identischen Betriebsbedingungen von jenen in B) I nicht unterscheidbar.

C) Heterogen katalysierte partielle Gasphasenoxidation von o-Xylol zu Phthalsäureanhydrid in einem Thermorohr

I. Beschickung eines Thermorohres mit einem ringförmige Schalenkatalysatoren umfassenden Katalysatorfestbett, wobei vorab der Einbringung der ringförmigen Schalenkatalysatoren eine Zwillingsabtrennung durchgeführt worden ist.

Das Thermorohr (V2A Stahl; Innendurchmesser: 25 mm, Wandstärke: 2 mm; Länge: 385 cm; in der Reaktionsrohrmitte zentriert eine Thermohülse (4 mm Außendurchmesser) zur Aufnahme eines einfachen, längs des Reaktionsrohres beweglichen Thermoelementes, um die Reaktionstemperatur längs des Reaktionsrohres zu ermitteln) wurde von oben nach unten wie folgt beschickt:

Abschnitt 1: 10 cm Länge Steatitringe S (Steatit C 220 der Fa. CeramTec) der Geometrie 5 mm x2mmx3mm(AxIxH);

Abschnitt 2: 170 cm Länge ringförmiger Schalenkatalysator gemäß Beispiel 7 der DE-A10344844 aber mit Zwillingsabtrennung (Suspensions-Aufsprüh-Verfahren; Trä- gergeometrie = 8 mm x 5 mm x 6 mm (A x I x H); Zwillingsabtrennung mittels eines Mogensen Sizer I; die Multimetalloxidaktivmassenschale enthielt 0,08 Gew.-% P; 5,75 Gew.-% Einheiten $V_2O_5$ 1,6 Gew.-% Einheiten $Sb_2O_3$; 0,4 Gew.-% Cs und 92,17 Gew.-% Einheiten $TiO_2$);

Abschnitt 3: 130 cm Länge ringförmiger Schalenkatalysator gemäß Beispiel 4 der DE-A10344844, aber mit Zwilingabtrennung (Suspensions-Aufsprüh-Verfahren; Trä- gergeometrie = 8 mm x 5 mm x 6 mm (A x I x H); Zwillingsabtrennung mittels Mogensen Sizer I; die Mulitmetalloxidaktivmassenschale ent- hielt 0,15 Gew.-% P; 7,5 Gew.-% Einheiten $V_2O_5$; 3,2 Gew.-% Einhei- ten $Sb_2O_3$ 0,1 Gew.-% Cs und 89,05 Gew.-% Einheiten $TiO_2$).

Das Thermorohr wurde von einem Salzbad (ein Gemisch aus 53 Gew.-% Kaliumnitrat, 40 Gew.-% Natriumnitrit und 7 Gew.-% Natriumnitrat umspült, das eine Temperatur von 350 °C aufwies

Das vorstehend beschriebene Thermorohr wurde mit einem Reaktionsgaseingangsgemisch aus 2 Vol.-% o-Xylol und 98 Vol.-% Luft beschickt.

Der Beschickungsgasstrom betrug 4 Nm³/h, bei einem Eingangsdruck von 1,4 bar (absolut). Das Reaktionsgasgemisch durchströmte das Thermorohr von oben nach unten. Bei einem auf den einmaligen Durchgang des Reaktionsgasgemischs durch das Thermorohr bezogenen Umsatz des o-Xylol von 99,97 mol-% betrug die Selektivität der Phthalsäureanhydridbildung 81,7 mol-%. Die Heißpunkttemperatur im Reaktionsrohr lag bei 452 °C. Die Position des Heißpunktmaximums war in Strömungsrichtung des Reaktionsgases 90 cm hinter dem Beginn des Abschnitt 2.

II. Wiederholung der Partialoxidation aus C) I, jedoch unter gezielter Dotierung des in das Thermorohr eingebrachten Katalysatorfestbetts mit einem bei der Herstellung der ringförmigen Schalenkatalysatoren aus Abschnitt 2 im Rahmen der Zwillingsabtrennung angefallenen Tandem-Zwilling (die Gesamtmenge der abgetrennten Zwillinge belief sich auf 0,3 Gew.-%)

Das Katalysatorfestbett wurde wie in C) I beschickt. In Strömungsrichtung des Reaktionsgases 90 cm hinter dem Beginn des Abschnitts 2 wurde zwischen die Innenwand des Thermorohres und die Außenwand der Thermohülse jedoch gezielt ein Abschnitt 2-Tandem-Zwilling 0,092 Gew.-% bezogen auf das Gesamtgewicht an ins Thermorohr insgesamt eingebrachten Schalenkatalysatoren) so platziert, dass seine Längsachse in die Strömungsrichtung des Reaktionsgases wies. Im übrigen wurde wie in C) I verfahren und die Thermorohrbefüllung auch demgemäß zu Ende geführt.

Bei unveränderter Position der Heißpunkttemperatur erhöhte sich diese auf 463 °C.

US Provisional Patent Application No. 60/944327, eingereicht am 15.06.07, ist eingefügt in die vorliegende Anmeldung durch Literaturhinweis. Im Hinblick auf die obengenannten Lehren sind zahlreiche Änderungen und Abweichungen von der vorliegenden Erfindung möglich. Man kann deshalb davon ausgehen, dass die Erfindung, im Rahmen der beigefügten Ansprüche, anders als hierin spezifisch beschrieben, ausgeführt werden kann.

**Patentansprüche**

1. Verfahren zum Einbringen einer wenigstens einer Produktionscharge von ringförmigen Schalenkatalysatoren K entnommenen Teilmenge in ein Reaktionsrohr eines Rohrbündelreaktors zum Zweck der Beschickung dieses Reaktionsrohres mit einem zur Durchführung einer heterogen katalysierten partiellen Gasphasenoxidation einer organischen Ausgangsverbindung geeigneten Katalysatorfestbett, **dadurch gekennzeichnet, dass** vorab der Entnahme der Teilmenge aus der wenigstens einen Produktionscharge und/oder nach der Entnahme aber vorab der Einbringung der entnommenen Teilmenge in das Reaktionsrohr bei der Herstellung der wenigstens einen Produktionscharge von ringförmigen Schalenkatalysatoren K entstandene Zwillinge von ringförmigen Schalenkatalysatoren K wenigstens teilweise aus der wenigstens einen Produktionscharge und/oder aus der entnommenen Teilmenge entfernt werden.

2. Verfahren nach Anspruch 1, **dadurch gekennzeichnet, dass** die Schalenkatalysatoren K aus einem ringförmigen Trägerformkörper und einer auf diesen aufgebrachten Schale aus Aktivmasse bestehen, wobei der Außendurchmesser des ringförmigen Trägerformkörpers 4 bis 10 mm, seine Höhe 2 bis 10 mm und seine Wanddicke 1 bis 4 mm betragen.

3. Verfahren nach Anspruch 2, **dadurch gekennzeichnet, dass** die Aktivmasse wenigstens ein Multielementoxid enthält.

4. Verfahren nach Anspruch 3, **dadurch gekennzeichnet, dass** das Multielementoxid ein solches ist, das

    a) die Elemente Mo, Fe und Bi, oder
    b) die Elemente Mo und V, oder
    c) das Element V sowie zusätzlich P und/oder Ti

enthält.

5. Verfahren nach einem der Ansprüche 1 bis 4, **dadurch gekennzeichnet, dass** die Entfernung der Zwillinge durch ein Verfahren der Siebung mit Hilfe eines Siebes erfolgt.

6. Verfahren nach Anspruch 5, **dadurch gekennzeichnet, dass** zwischen dem Außendurchmesser A und der Höhe H des ringförmigen Schalenkatalysators K die Beziehung $H \leq 0,5 \cdot A$ erfüllt ist und das Sieb Sieböffnungen $O_1$ aufweist, in deren geschlossene Umrisslinie ein Rechteck R mit den Seitenlängen L und C zwar unter der Maßgabe $M_1$,

$$L > A \geq 2\,H > C > H,$$

aber nicht mehr unter der Maßgabe $M_1^*$,

$$L > C \geq 2\,H$$

einbeschrieben werden kann.

7. Verfahren nach Anspruch 5, **dadurch gekennzeichnet, dass** zwischen dem Außendurchmesser A und der Höhe H des ringförmigen Schalenkatalysators K die Beziehungen $A \geq H > 0,5 \cdot A$ erfüllt sind und das Sieb Sieböffnungen $O_3$ aufweist, in deren geschlossene Umrisslinie ein Rechteck R mit den Seitenlängen L und C zwar unter Maßgabe $M_3$,

$$L > A < 2 H > C > H,$$

aber nicht mehr unter der Maßgabe M3*,

$$L \geq C \geq 2 H$$

einbeschrieben werden kann.

8.  Verfahren nach Anspruch 6, **dadurch gekennzeichnet, dass** die Sieböffnung $O_1$ ein Rechteck mit den Seitenlängen L und C ist.

9.  Verfahren nach Anspruch 7, **dadurch gekennzeichnet, dass** die Sieböffnung $O_3$ ein Rechteck mit den Seitenlängen L und C ist.

10. Verfahren nach Anspruch 6, **dadurch gekennzeichnet, dass** die Sieböffnung $O_1$ ein Langloch ist, das sich von einem Rechteck mit den Seitenlängen L und C ableitet.

11. Verfahren nach Anspruch 7, **dadurch gekennzeichnet, dass** die Sieböffnung $O_3$ ein Langloch ist, das sich von einem Rechteck mit den Seitenlängen L und C ableitet.

12. Verfahren nach einem der Ansprüche 1 bis 11, **dadurch gekennzeichnet, dass** sich ein Verfahren der heterogen katalysierten partiellen Gasphasenoxidation einer organischen Ausgangsverbindung im mit dem Katalysatorfestbett beschickten Reaktionsrohr anschließt.

13. Verfahren nach Anspruch 12, **dadurch gekennzeichnet, dass** die heterogen katalysierte partielle Gasphasenoxidation die Gasphasenoxidation von Acrolein zu Acrylsäure oder die Gasphasenoxidation von o-Xylol und/oder Naphthalin zu Phthalsäureanhydrid ist.

14. Verfahren zur Herstellung von ringförmigen Schalenkatalysatoren durch Aufbringen einer katalytischen Aktivmasse auf ringförmige Trägerformkörper mit Hilfe eines flüssigen Bindemittels, **dadurch gekennzeichnet, dass** nach Beendigung des Herstellverfahrens bei der Herstellung entstandene Schalenkatalysatorringzwillinge wenigstens teilweise abgetrennt werden.

15. Verwendung von ringförmigen Schalenkatalysatoren, die nach einem Herstellverfahren gemäß Anspruch 14 hergestellt worden sind zum Beschicken von Reaktionsrohren in einem Rohrbündelreaktor mit einem Katalysatorfestbett.

16. Durch Umhüllen einer Mengenportion von Katalysatorformkörpern mit einem Verpackungsmittel erzeugte Packung, **dadurch gekennzeichnet, dass** die Katalysatorformkörper ringförmige Schalenkatalysatoren umfassen, die nach einem Verfahren gemäß Anspruch 14 hergestellt worden sind.

**Claims**

1.  A process for introducing a portion withdrawn from at least one production charge of annular coated catalysts K into a reaction tube of a tube bundle reactor for the purpose of charting this reaction tube with a fixed catalyst bed suitable for performing a heterogeneously catalyzed partial gas phase oxidation of an organic starting compound, which comprises, before the withdrawal of the portion from the at least one production charge and/or after the withdrawal but before the introduction of the portion withdrawn into the reaction tube, removing adhering pairs of annular coated catalysts K formed in the preparation of the at least one production charge of annular coated catalysts K at least partly from the at least one production charge and/or from the portion withdrawn.

2.  The process according to claim 1, wherein the coated catalysts K consist of an annular shaped support body and a coasting of active composition applied thereto, the external diameter of the annular shaped support body being from 4 to 10 mm, its height from 2 to 10 mm and its wall thickness from 1 to 4 mm.

3. The process according to claim 2, wherein the active composition comprises at least one multielement oxide.

4. The process according to claim 3, wherein the multielement oxide is one which comprises

  a) the elements Mo, Fe and Bi, or
  b) the elements. Mo and V, or
  c) the element V and additionally P and/or Ti.

5. The process according to any of claims 1 to 4, wherein the adhering pairs are removed by a process for screening with the aid of a screen.

6. The process according to claim 5, wherein, between the external diameter E and the height H of the annular coated catalyst K, the relationship $H \leq 0.5 \cdot E$ is satisfied and the screen has screen orifices $O_1$ within whose continuous outline a rectangle R with the side lengths L and C can be inscribed with the proviso $M_1$,

$$L > E \geq 2H > C > H,$$

but not with the proviso $M_1^*$.

$$L > C \geq 2H.$$

7. The process according to claim 5, wherein, between the external diameter E and the height H of the annular coated catalyst K, the relationship $E \geq H > 0.5 \cdot E$ is satisfied and the screen has screen orifices $O_3$ within whose continuous outline a rectangle R with the side lengths L and C can be inscribed with the proviso $M_3$,

$$L > E < 2H > C > H,$$

but not with the proviso $M_3^*$,

$$L \geq C \geq 2H.$$

8. The process according to claim 6, wherein the screen orifice $O_1$ is a rectangle with the side lengths L and C.

9. The process according to claim 7, wherein the screen orifice $O_3$ is a rectangle with the side lengths L and C.

10. The process according to claim 6, wherein the screen orifice $O_1$ is an elongated hole which derives from a rectangle with the side lengths L and C.

11. The process according to claim 7, wherein the screen orifice $O_3$ is an elongated hole which derives from a rectangle with the side lengths L and C.

12. The process according to any of claims 1 to 11, which is followed by a process for heterogeneously catalyzed partial gas phase oxidation of an organic starting compound in the reaction tube charged with the fixed catalyst bed.

13. The process according to claim 12, wherein the heterogeneously catalyzed partial gas phase oxidation is the gas phase oxidation of acrolein to acrylic acid or the gas phase oxidation of o-xylene and/or naphtalene to phthalic anhydride.

**14.** A process for preparing annular coated catalysts by applying a catalytic active composition to annular shaped support bodies with the aid of a liquid binder, which comprises, after the preparation process has ended, at least partly removing adhering pairs of coated catalyst rings formed in the preparation.

**15.** The use of annular coated catalysts which have been prepared by a preparation process according to claim 14 for charging reaction tubes in a tube bundle reactor with a fixed catalyst bed.

**16.** A packing obtained by enveloping a portion of shaped catalyst bodies with a packing medium, wherein the shaped catalyst bodies comprise annular coated catalysts which have been prepared by a process according to claim 14.

## Revendications

**1.** Procédé d'introduction d'une partie prélevée d'au moins une charge de production de catalyseurs à enveloppe annulaires K dans un tube de réaction d'un réacteur à faisceau de tubes afin de charger ce tube de réaction avec un lit catalytique fixe approprié pour la réalisation d'une oxydation partielle en phase gazeuse sous catalyse hétérogène d'un composé de départ organique, **caractérisé en ce qu'**avant le prélèvement de la partie de la ou des charges de production et/ou après le prélèvement mais avant l'introduction de la partie prélevée dans le tube de réaction, des jumeaux de catalyseurs à enveloppe annulaires K formés lors de la fabrication de la ou des charges de production de catalyseurs à enveloppe annulaires sont éliminés au moins en partie de la ou des charges de production et/ou de la partie prélevée.

**2.** Procédé selon la revendication 1, **caractérisé en ce que** les catalyseurs à enveloppe K sont constitués d'un corps moulé support annulaire et d'une enveloppe de masse active appliquée sur celui-ci, le diamètre extérieur du corps moulé support annulaire étant de 4 à 10 mm, sa hauteur de 2 à 10 mm et son épaisseur de paroi de 1 à 4 mm.

**3.** Procédé selon la revendication 2, **caractérisé en ce que** la masse active contient au moins un oxyde de plusieurs éléments.

**4.** Procédé selon la revendication 3, **caractérisé en ce que** l'oxyde de plusieurs éléments est tel qu'il contient

    a) les éléments Mo, Fe et Bi, ou
    b) les éléments Mo et V, ou
    c) l'élément V et également P et/ou Ti.

**5.** Procédé selon l'une quelconque des revendications 1 à 4, **caractérisé en ce que** l'élimination des jumeaux a lieu par un procédé de tamisage à l'aide d'un tamis.

**6.** Procédé selon la revendication 5, **caractérisé en ce que** la relation $H \leq 0,5 \cdot A$ est remplie entre le diamètre extérieur A et la hauteur H du catalyseur à enveloppe annulaire K et le tamis présente des ouvertures de tamisage $O_1$, dans le contour fermé desquelles un rectangle R de côtés L et C peut être inscrit, sous la condition $M_1$

$$L > A \geq 2H > C > H$$

mais plus sous la condition $M_1^*$

$$L > C \geq 2H.$$

**7.** Procédé selon la revendication 5, **caractérisé en ce que** la relation $A \geq H > 0,5 \cdot A$ est remplie entre le diamètre extérieur A et la hauteur H du catalyseur à enveloppe annulaire K et le tamis présente des ouvertures de tamisage $O_3$, dans le contour fermé desquelles un rectangle R de côtés L et C peut être inscrit, sous la condition $M_3$

$$L > A < 2H > C > H$$

mais plus sous la condition $M_3^*$

$$L \geqq C \geqq 2H.$$

8. Procédé selon la revendication 6, **caractérisé en ce que** l'ouverture de tamisage $O_1$ est un rectangle de côtés L et C.

9. Procédé selon la revendication 7, **caractérisé en ce que** l'ouverture de tamisage $O_3$ est un rectangle de côtés L et C.

10. Procédé selon la revendication 6, **caractérisé en ce que** l'ouverture de tamisage $O_1$ est une fente allongée qui dérive d'un rectangle de côtés L et C.

11. Procédé selon la revendication 7, **caractérisé en ce que** l'ouverture de tamisage $O_3$ est une fente allongée qui dérive d'un rectangle de côtés L et C.

12. Procédé selon l'une quelconque des revendications 1 à 11, **caractérisé en ce qu'**un procédé d'oxydation partielle en phase gazeuse sous catalyse hétérogène d'un composé de départ organique s'ensuit dans le tube de réaction chargé avec le lit catalytique fixe.

13. Procédé selon la revendication 12, **caractérisé en ce que** l'oxydation partielle en phase gazeuse sous catalyse hétérogène est l'oxydation en phase gazeuse d'acroléine en acide acrylique ou l'oxydation en phase gazeuse d'o-xylène et/ou de naphtaline en anhydride de l'acide phtalique.

14. Procédé de fabrication de catalyseurs à enveloppe annulaires par application d'une masse active catalytique sur des corps moulés supports annulaires à l'aide d'un liant liquide, **caractérisé en ce que** des anneaux jumeaux de catalyseur à enveloppe formés lors de la fabrication sont éliminés au moins en partie après la fin du procédé de fabrication.

15. Utilisation de catalyseurs à enveloppe annulaires qui ont été fabriqués par un procédé de fabrication selon la revendication 14 pour le chargement de tubes de réaction dans un réacteur à faisceau de tubes avec un lit catalytique fixe.

16. Garnissage formé par enveloppement d'une proportion de corps moulés catalytiques avec un agent d'emballage, **caractérisé en ce que** les corps moulés catalytiques comprennent des catalyseurs à enveloppe annulaires qui ont été fabriqués par un procédé selon la revendication 14.

Figur 1

Figur 2

Figur 3

Figur 4

Figur 5

Figur 6

Figur 7

Figur 8

Figur 9

Figur 10

Figur 11

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- CH 449600 A
- CH 38828 A
- DE 2351151 A
- DE 2526238 A
- EP 092097 A
- EP 058927 A
- DE 4132263 A
- DE 4132684 A
- DE 4022212 A
- EP 522871 A
- DE 2106796 A
- DE 1624921 A
- GB 1464198 A
- GB 1291354 A
- DE 2025430 A
- EP 352849 A
- EP 352850 A
- EP 532325 A
- US 5155242 A
- US 5262551 A
- DE AS1254137 B
- DE 2159346 A
- EP 372972 A
- WO 8907101 A
- DE 4311608 A
- DE 10131297 A
- EP 1090684 A
- EP 608838 A
- DE 10046672 A
- EP 529853 A
- WO 0196270 A
- DE 10028582 A
- DE 202006014116 U1
- EP 700893 A
- DE 4431949 A
- WO 03057653 A
- EP 1695954 A
- WO 03055835 A
- WO 03059857 A
- WO 03076373 A
- DE 69915952 T2
- DE 102004018267 A
- DE 102007019597
- EP 1734030 A
- DE 10313214 A
- DE 10313219 A
- DE 10313211 A
- DE 10313208 A
- EP 1388533 A

- US 6069271 A
- EP 990636 A
- US 20060161019 A
- EP 1106598 A
- EP 873783 A
- EP 1270065 A
- DE 10350822 A
- DE 102004025445 A
- DE 10351269 A
- DE 10232748 A
- DE 102007010422 A
- EP 1471046 A
- DE 202006014116U1 A
- JP 2006142288 A
- US 4701101 A
- EP 1466883 A
- US 2006245992 A
- US 2002136678 A
- WO 2005051532 A
- JP 2004195279 A
- WO 200503039 A
- DE 102005010645 A
- DE 2909671 A
- EP 714700 A
- DE 102007017080
- WO 2004108267 A
- DE 102005010645 A1
- DE 10313209 A
- DE 10325488 A
- DE 10360058 A
- WO 2007009922 A
- DE 10049873 A
- DE 102007010422
- DE 4006935 A
- DE 19823275 A
- DE 19839001 A
- DE 19823262 A
- DE 10344844 A
- US 20060205978 A
- EP 758562 A
- DE 102004023249 A
- EP 1726358 A
- DE 3520614 A
- EP 205089 A
- DE 3431337 A
- US 7147011 A

- WO 200700922 A
- DE 102007025869 A
- EP 496470 A
- EP 619142 A
- EP 624398 A
- EP 804289 A
- EP 937498 A

- US 6528683 A
- US 6586361 A
- US 6362345 A
- DE 102005055827 A
- DE 0344844 A1
- US 60944327 B


**In der Beschreibung aufgeführte Nicht-Patentliteratur**

- *Aufbereitungs-Technik-Nr. 11/1960,* 1960, 457-473
- *Chem.-Ing.-Techn. 56,* 1984, 897-907
- **Paul Schmidt et al.** Sieben und Siebmaschinen. Wiley-VCH GmbH & Co, 2003
- *Chem.-Ing.-Tech.,* 1984, vol. 56 (12), 987-907

- **Paul Schmidt.** Grundlagen und Anwendung. Wiley VCH, 2003
- *Aufbereitungstechnik,* 2001, vol. 42 (7), 345-348
- *Aufbereitungstechnik,* 2000, vol. 41 (7), 325-329
- *Chem.-Ing.Tech.,* 1984, vol. 56 (12), 902